# EUROPEAN PATENT APPLICATION

(11) **EP 1 348 758 A1**
(43) Date of publication of application: **01.10.2003**
(21) Application number: 02007144.5
(22) Date of filing: 28.03.2002
(51) Int. Cl.: C12N 5/06

(54) **Host cells having improved cell survival properties and methods to generate such cells**

(71) Applicant: Boehringer Ingelheim Pharma GmbH & Co.KG, 55218 Ingelheim am Rhein (DE)
(72) Inventor: Enenkel, Barbara, 88447 Warthausen (DE); Meents, Heiko, 79415 Bad Bellingen (DE); Fussenegger, Martin, 8046 Zürich (CH)

(57) **Abstract**

The present invention relates to genetically engineered mammalian host cells comprising an enhanced level of active anti-apoptosis genes and methods to generate such host cells. More particularly, the invention pertains to methods which modulate the level of anti-apoptosis active genes within host cells and to host cells showing an enhanced cell viability by delaying/inhibiting programmed cell death naturally occurring in such cells.

## Description

### FIELD OF THE INVENTION

The present invention relates to genetically engineered mammalian host cells comprising an enhanced level of active anti-apoptosis genes and methods to generate such host cells. More particularly, the invention pertains to methods which modulate the level of anti-apoptosis active genes within host cells and to host cells showing an enhanced cell viability by delaying/ inhibiting programmed cell death naturally occurring in such cells.

### BACKGROUND OF THE INVENTION

Mammalian cells are the preferred host for the production of most complex protein therapeutics, as functionally and pharmacokinetically relevant post-translational modifications are highly human-compatible. Commercially relevant cell types include hybridomas, myelomas, chinese hamster ovary (CHO) cells and baby hamster kidney (BHK) cells. CHO derivatives are being increasingly used in industry due to their straightforward adaptation for growth in serum-/protein-free media and their consideration as safe production hosts by key regulatory agencies.

During the standard biopharmaceutically production processes - bioreactor operations - a substantial percentage of the production cell population die following a genetically determined program known as apoptosis (Al-Rubeai et al., 1998; Goswami et al., 1999; Lakenet al., 2001). Apoptosis is known as an active cellular suicide program activated as a result of either extrinsic or intrinsic signals, such as serum deprivation, nutrient limitation, oxygen limitation and mechanical stress. Apoptosis is characterized by plasma membrane blebbing, cell volume loss, nuclear condensation, and endonuleolytic degradation of DNA at nucleosomal intervals (Wyllie et al., 1980).

Serum components have been identified as major effective apoptosis-protective agents. However, there is a strong drive within the biotechnology industry, also pushed by the key regulatory agencies for drug registration, towards the development of serum-free and protein-free manufacturing processes. The main reasons are reduction of costs, less interference during protein purification, and reduction of the potential for introduction of pathogens, such as prions or viruses. However, omission of serum often leads to an increased sensitivity of production cell lines to programmed cell death, making cells more vulnerable to cultural insults (Franek et al., 1991; Goswami et al., 1999; Moore et al., 1995; Zanghi et al., 1999). Premature cell death, e.g. by apoptosis, represents a high loss of valuable resources, because protein production is primarily a function of the viable producing cell population. The longer a high density of viable and productive cells in culture can be maintained, the more effective and profitable is the production process due to higher product yields per production run. In the absence of serum the cells die more rapidly at reaching the stationary growth phase at maximal cell density, thus cutting the production phase short. Yields are even further reduced by product degradation by proteases released from apoptotic cells and interference during the harvest caused by high numbers of cell debris.

A variety of physiological engineering strategies have been initiated to solve the dilemma of the increased apoptosis sensitivity of production cells lines, particularly of those cell lines being adapted and completely cultivated under serum-free conditions. Some of those strategies are focused at the reduction/elimination of the induction of endogenous apoptosis-response programs under production-mimicking cell culture conditions. One approach is directed on alleviating nutrient deprivation by feeding (deZengotita et al., 2000; Franek et al., 1996; Mercille et al., 1994; Sanfeliu et al., 1999). Another method is focussed on the use of apoptosis-suppressing chemical additives for blocking key effectors of apoptosis response mechanisms (Mastrangelo et al., 1999; Sanfeliu et al., 2000; Simpson et al., 1998; Zanghi et al., 2000). An alternative strategy represents a genetic approach. It includes the manipulation of the cell itself using anti-apoptotic survival genes or engineered anti-apoptosis determinants derived from survival-maintaining regulatory networks or viruses (Cotter et al., 1995; Chung et al., 1998; Fussenegger et al., 2000; Mastrangelo et al., 1998, 2000a and 2000b; Mercille et al., 1999a and 1999b; Pan et al., 1998; Simpson et al., 1999; Terada et al., 1997; Tey et al., 2000a and 2000b).

Of the latter strategy bcl-2 and bcl-xL, two apoptosis suppressors and key members of a family of highly conserved pro- (for example bad, bak, bax, bok, bcl-xS, bik, bid, hrk, bim, blk, bcl-y) and anti-apoptotic (for example bcl-2, bcl-xL, blc-w, bfl-1, al, mcl-1, boo, brag-1, nr-13, bhrf-1, ced 9, cdn-1, cdn-2, cdn-3) response regulator genes, have been postulated to be potent candidates for anti-apoptosis engineering in the biotech community.

Bcl-2 has been shown to modulate induction of caspase-9-dependent apoptosis pathway at the outer membrane of mitochondria in response to a molecular rheostat localized at the outer mitochondrial membrane consisting of homo- and heterodimerized pro- and anti-apoptotic BCL-2-type proteins. Biased expression towards a pro-apoptotic subfamily member by culture constraints and endogenous signals results in release of Apaf 1-caspase-9 complex and auto-activation of this cysteine-containing aspartate-specific protease correlating with dramatic cytochrome c efflux from mitochondria into the cytosol. Assembly of major parts of the apoptosis-controlling machinery in the outer mitochondrial membrane bring these cellular power stations, acting as stress sensors and executioners for maintenance of the apoptosis process, in the focus of anti-apoptosis engineering (Follstad et al., 2000; Green et al., 1998; Tsujimoto, 1998).

There are some reports on the positive effect of expression of a heterologous anti-apoptosis bcl-2 gene on survival/viability and robustness of engineered cell lines, adapted on and cultivated in presence of calf serum. Those cells include myelomas, hybridomas, baby hamster kidney cells (BHK), and chinese hamster ovary cells (CHO). The positive effect of BCL-2 expression has been described in connection with various environmental insults like serum and glucose deprivation, growth factor withdrawal or viral infections (Fassnacht et al., 1998; Figueroa et al., 2001; Fussenegger et al., 2000; Itoh et al., 1995; Mastrangelo et al., 2000a and 2000b; Reynolds et al., 1996; Simpson et al., 1997, 1998 and 1999; Tey et al., 2000a and 2000b)

BCL-xL-based metabolic engineering has been described for human T cells and CD34⁺ hematopoietic cells, respectively (US patent No. 6,143,291, WO 00/75291). Fussenegger et al. al., 1998 and also Mastrangelo et al., 2000a and 2000b have described a positive effect of BCL-xL expression on survival of hamster cells.

However, in all those reports either non-production cell lines such as human T cells and CD34⁺ hematopoietic cells were used, or if production cell lines, e.g. hamster or mice cell lines, were used, they were adapted and routinely grown in the presence of serum and in most cases even as adherent cells. Studies on the effect of serum withdrawal on the survival of recombinant cells expressing heterologous BCL-2 or BCL-xL did not include cultivation for several passages in serum-free medium. Rather, senim-cultivated cells were either seeded into completely serum-free medium or medium with reduced serum content and the effect of this cultural insult on cell survival in batch culture was followed for several days. This way the actual onset of cultural insult caused by serum withdrawal might also be delayed due to still ongoing intracellular reactions triggered by apoptosis-protective agents within the serum.

Only few experiments have been performed on production relevant recombinant cell lines adapted on and constantly grown in suspension in serum-free medium. These conditions are most favored in biotechnology industry nowadays, but at the same time cells are also more fragile and less robust. In the publication of Goswami et al., 1999, experiments are described in which CHO cells expressing γ-interferon were cultivated under those conditions. Survival of cultures expressing in addition heterologous BCL-2 were improved but limited to about 40% viable cells after day 7 in a batch culture. Nothing is known about the effect of BCL-xL expression on viability/survival of host cells, adapted and permanently grown under serum-free conditions.

From the above discussion, it is apparent that there is a need in the biotech community for further increasing cell viability, especially of cell lines adapted to serum free growth and qualified for serum free production of biopharmaceuticals for therapeutic and diagnostic use. The extension of cell survival at high viabilities as the cells reach the stationary phase in batch cultures would significantly influence productivity and cost-effectiveness, whereby every additional day gained during the production phase would make a great contribution.

In the present invention strategies are provided to further improve the survival of production cell lines grown constantly in suspension in serum-free medium. The strategies are based on engineering host cells in order to improve the intracellular-level of anti-apoptotic acting polypeptides. It has been surprisingly found, that the level of intracellular anti-apoptotic acting genes can substantially improve cell viability without showing any negative effect on cell productivity.

### SUMMARY OF THE INVENTION

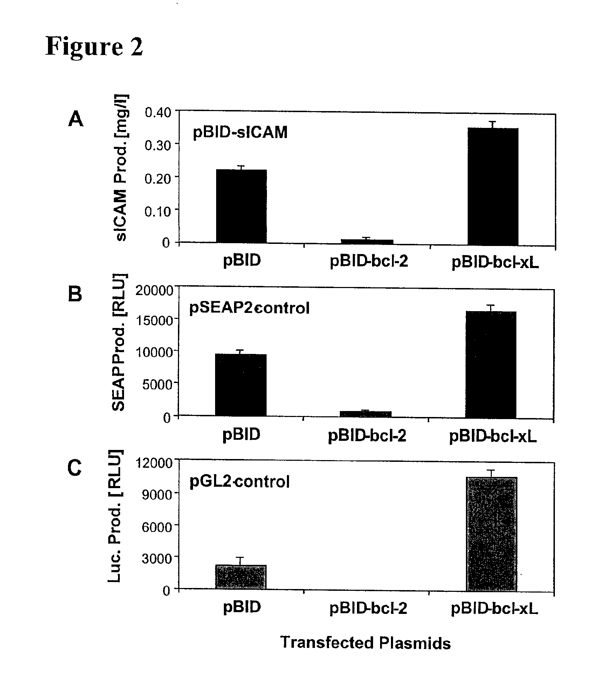

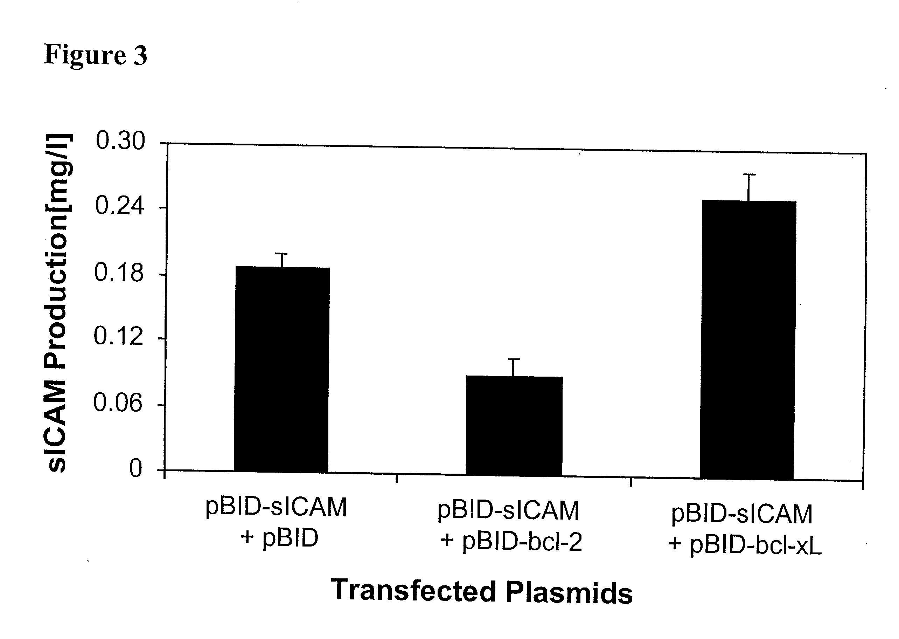

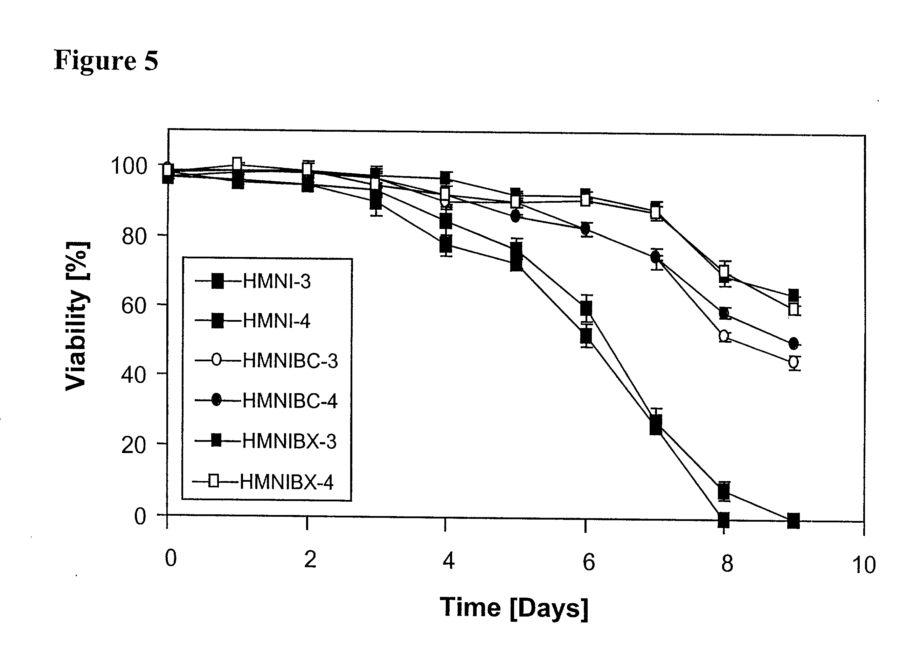

It has been demonstrated, that the expression of a non-amplified heterologous introduced anti-apoptosis gene, e.g. BCL-2 or BCL-xL has only a minor effect on the viability/survival of cells adapted and generally grown under serum- and/or protein-free condition (see Figure 5). This finding is supported by the publication of Goswami et al., 1999. The present invention describes/provides now a new approach to dramatically improve the viability/survival of host cells, particularly when established and cultivated under serum-free conditions. Apoptosis delay/inhibition is achieved by improving the intracellular level of anti-apoptotic acting proteins, e.g. BCL-xL or BCL-2, using amplification-mediated over-expression. It has been surprisingly found by the present invention, that an enormous over-expression of an anti-apoptosis protein such as BCL-xL is a more than suitable tool to further improve cell viability/survival, without negatively affecting the expression level of a desired protein. On the contrary, cell productivity in BCL-xL over-expressing cells is enhanced (for example see Figure 2 and 3). Therefore, the present invention provides a person skilled in the art with new genetically modified host cells, in particular hamster or murine production cell lines, and also with methods to generate such inventive cell lines. This finding is highly advantageous for the production of biopharmaceutical peptides/proteins as now the economic viability of production processes can be improved in a very sufficient manner. A great advantage of the present invention over the art consists in that methods are provided which connect improvement of cell viability, a limiting factor in protein production processes, with the high yield protein production.

The present invention therefore provides genetically engineered host cells having improved survival properties and being highly suitable for the production of biopharmaceutical proteins. The improved survival properties are based on an enhanced level of active anti-apoptosis genes within the cells. Preferred are host cells of mammalian origin, more preferred murine hybridoma/myelomas or hamster cells, especially if adapted and constantly grown in serum-free and/or protein-free media.

The present invention also provides a method of generating mammalian host cells showing such an enhanced expression level of an anti-apoptosis gene, comprising (i.) introducing into a mammalian cell population nucleic acid sequences that encode for an anti-apoptosis gene, a selectable amplifiable marker gene, and optionally at least one gene of interest, wherein said genes are operatively linked to at least one regulatory sequence allowing for expression of said genes, (*ii*.) cultivating said cell population under conditions where at least said selectable amplifiable marker gene and said anti-apoptosis gene are expressed, and which are favourable for obtaining multiple copies at least of the anti-apoptosis gene, and (*iii*.) selecting cells from the cell population that incorporate multiple copies at least of the anti-apoptosis gene.

The present invention, therefore, also provides methods of expressing an anti-apoptosis gene, an selectable amplifiable marker gene and at least one gene of interest in a host cell, comprising (*i*.) introducing into a host cell population the nucleic acid sequences that encode for an anti-apoptosis gene, a selectable amplifiable marker gene, and a gene(s) of interest, wherein said genes are operatively linked to at least one regulatory sequence allowing for expression of said genes, and (*ii*.) cultivating said host cell population under conditions wherein said genes are expressed.

Manipulation of host cells by any of these methods provides host cells comprising at least a high copy number of an anti-apoptosis gene.

Finally, the present invention also provides processes for producing a protein of interest in any host cell according to this invention, comprising (i.) cultivating cells under conditions which are favourable for the expression of the anti-apoptosis gene and the gene(s) of interest and (*ii*.) isolating the protein of interest from the cells and/or the cell culture supernatant.

### BRIEF DESCRIPTION OF THE DRAWINGS

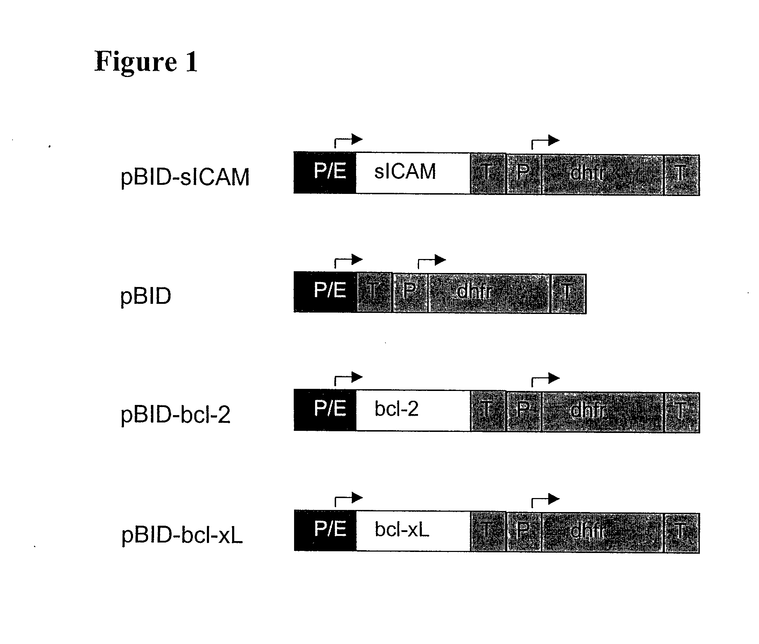

Figure 1 schematically shows the expression vector designs used for the transfection of CHO-DG44 cells. P/E means a composite unit that contains both enhancer and promoter element, P a promoter element and T a transcription termination site required for polyadenylation of transcribed messenger RNA. sICAM refers to the gene of interest and bcl-2 or bcl-xl to the anti-apoptosis genes. dhfr refers to the amplifiable selectable marker dihydrofolate reductase. An arrow indicates the site of transcription initiation within a transcription unit.
Figure 2 compares the expression profiles of transiently transfected CHO-DG44 cells 48 hours post transfection. In Figure 2A pBID-sICAM, in Figure 2B pSEAP2-Control (Clontech, Palo Alto, CA) and in Figure 2C pGL2-Control (Promega, Madison, WI) was cotransfected with either pBID-bcl-2, pBID-bcl-xL or the control pBID.
Figure 3 shows sICAM expression profiles of stable mixed populations of CHO-DG44 cells cotransfected with pBID-sICAM and either pBID, pBID-bcl-2 or pBID-bcl-xL, selected and cultivated in hypoxanthine/thymidine free CHO-S-SFMII medium (Invitrogen, Carlsbad, CA). Each value resulted from an average sICAM production of six mixed populations during a 3 day culture period over 5 passages.

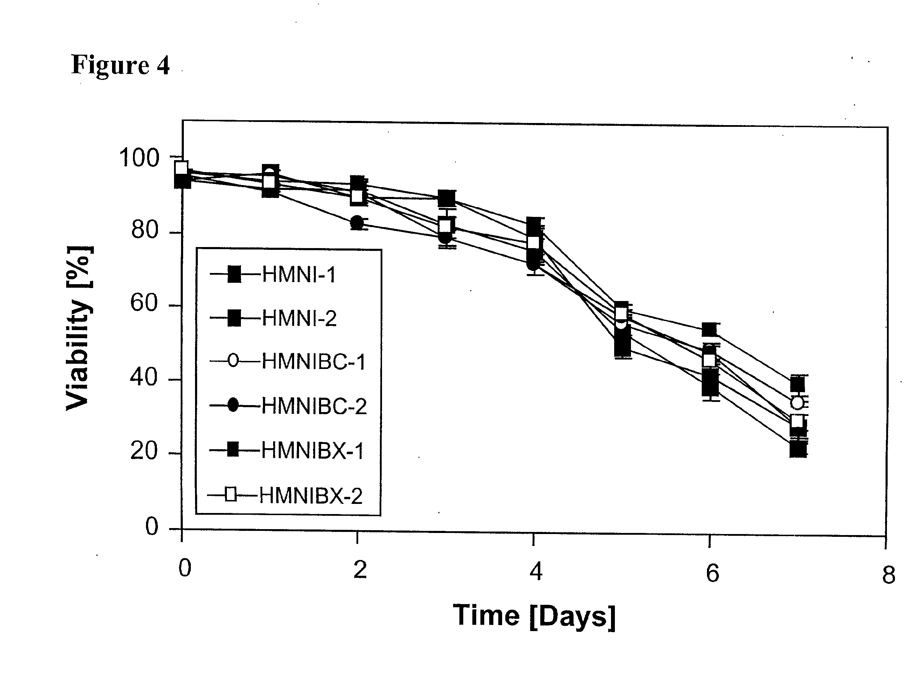

Figure 4 assesses the viability of clonal cell lines HMNI-1/2 (control cell lines cotransfected with pBID-sICAM and pBID), HMNIBC-1/2 (cotransfected with pBID-sICAM and pBID-bcl-2) and HMNIBX-1/2 (cotransfected with pBID-sICAM and pBID-bcl-xL) during a 7-day batch cultivation period using trypan blue dye exclusion. The recombinant CHO-DG44 cells were selected and cultivated in hypoxanthine/thymidine free CHO-S-SFMII medium (Invitrogen, Carlsbad, CA).
Figure 5 shows the percent viability profiles, using trypan blue dye exclusion, of methotrexate-amplified cell clones HMNI-3/4 (control cell lines cotransfected with pBID-sICAM and pBID), HMNIBC-3/4 (cotransfected with pBID-sICAM and pBID-bcl-2) and HMNIBX-3/4 (cotransfected with pBID-sICAM and pBID-bcl-xL) during a 9-day batch cultivation in hypoxanthine/thymidine free CHO-S-SFMII medium (Invitrogen, Carlsbad, CA) and the presence of 20 nM MTX.

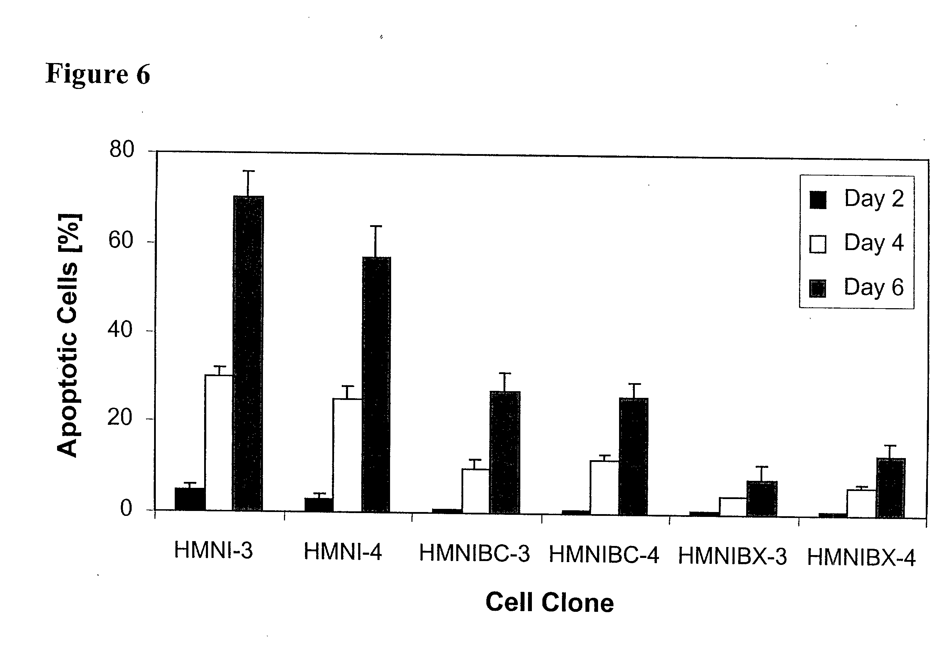

Figure 6 shows the apoptosis characteristics of methotrexate-amplified CHO-DG44 cell clones expressing BCL-2 or BCL-xL. Percentage of apoptotic cells among HMNI-3/4 (control cell lines cotransfected with pBID-sICAM and pBID), HMNIBC-3/4 (cotransfected with pBID-sICAM and pBID-bcl-2) and HMNIBX-3/4 (cotransfected with pBID-sICAM and pBID-bcl-xL) batch cultures in hypoxanthine/thymidine free CHO-S-SFMII medium (Invitrogen, Carlsbad, CA) plus 20 nM MTX were assessed at days 2, 4 and 6 using a fluorescence-based TUNEL assay (BD Biosciences PharMingen, San Diego,CA).

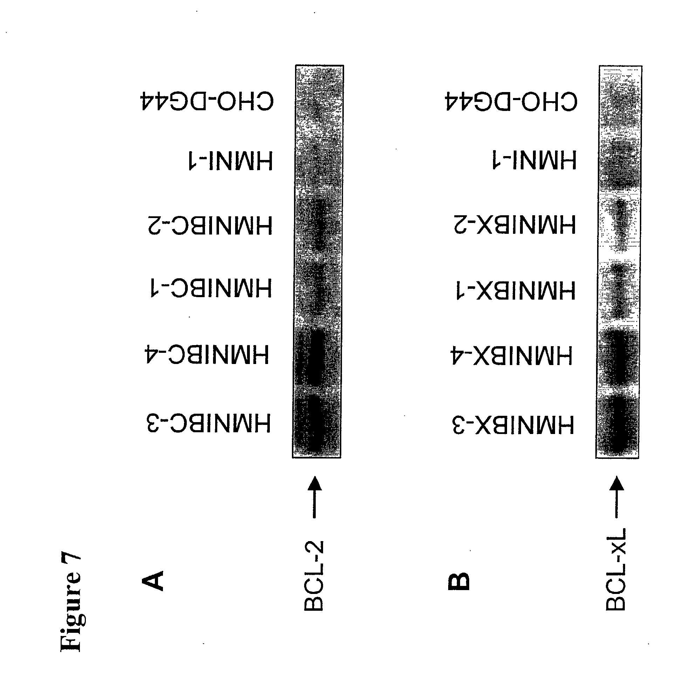

Figure 7 shows the Western blot analysis of BCL-2 and BCL-xL in different recombinant CHO-DG44 cell clones. It compares BCL-2 (Fig. 7A) and BCL-xL (Fig. 7B) expression in either unamplified (BCL-2: HMNIBC-1/2; BCL-xL: HMNIBX-1/2) or amplified (BCL-2: HMNIBC-3/4; BCL-xL: HMNIBX-3/4) cell clones expressing the anti-apoptosis genes in monocistronic configuration. Anti-apoptosis gene expression was compared to the parental (CHO-DG44) and sICAM-only producing (HMNI-1) control cell lines. Proteins were detected using mouse monoclonal antibodies from Santa Cruz Biotechnology (Santa Cruz, CA) specific for BCL-2 or BCL-xL and an anti-mouse peroxidase-coupled secondary antibody (Dianova GmbH, Hamburg, Germany).

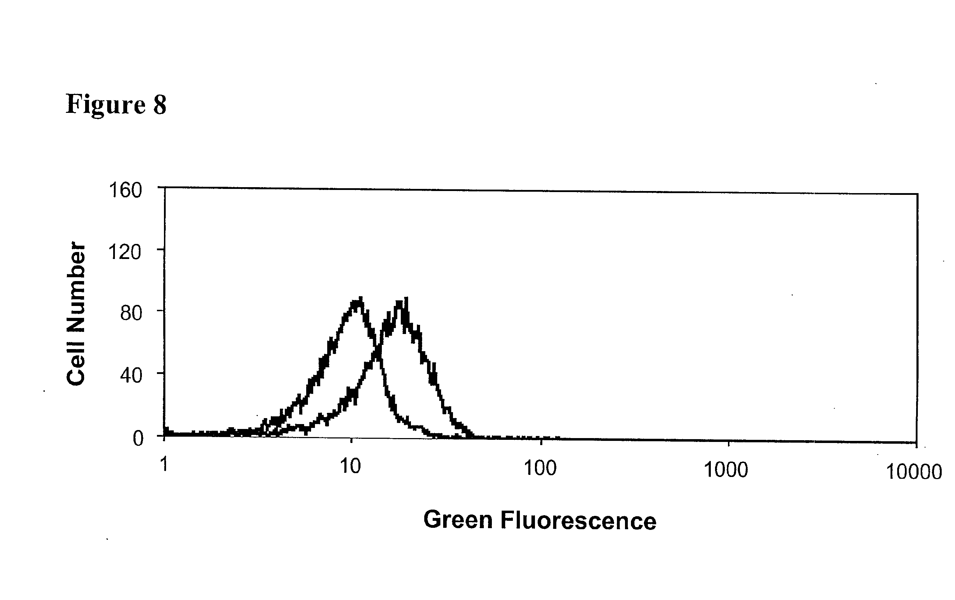

Figure 8 shows the MitoTracker-based quantification (Molecular Probes, Eugene, OR) of the mitochondria content in CHO-DG44 cells grown in the presence and absence of serum. FACS-mediated mitochondria counts were performed on CHO-DG44 cells cultivated in the presence (grey line) of 10% serum and in the absence (black line) of serum.

### DETAILED DESCRIPTION OF THE INVENTION

The present invention provides host cells which are genetically modified by introducing nucleic acid sequences that encode for an anti-apoptotic gene, a selectable amplifiable marker gene, and at least one gene of interest. In a preferred embodiment the anti-apoptosis gene, the selectable amplifiable marker gene and the gene(s) of interest are operatively linked to at least one regulatory sequence allowing for expression of said genes. In a further embodiment, the genetic modification of host cells includes the introduction of more than one anti-apoptosis gene.

Host cells modified in this manner allow co-expression of the anti-apoptosis gene(s) together with an selectable amplifiable marker, and optionally with a gene of interest. The selectable amplifiable marker not only enables selection of stable transfected host cell clones but also amplification of the anti-apoptosis gene(s). It is shown by the current invention that amplification of an anti-apoptosis gene provides a more efficient method for achieving intracellular levels of anti-apoptotic acting proteins, which are sufficient to improve survival of the host cells compared to host cells without any amplified anti-apoptosis protein encoding sequence.

Genetically modified host cells according to this invention are characterized by an enhanced cell survival attributed to an delayed or inhibited programmed cell death, e.g. apoptosis, within the host cells. It has been surprisingly found that a population of host cells modified by any method of this invention are being cultivable for at least 9 days, wherein the total viability of cells is at least about 50% (see also Figure 5). After a 8-day batch cultivation viability of at least about 60% is achievable by host cells generated according to this invention. In a further embodiment of this invention, cell viability of at least about 75% is obtainable for a 7-day batch cultivation. In another embodiment, after a 6-day cultivation period cell viability of at least about 85% can be achieved. Equivalent levels of viability are not known in the art, at least not for serum free cultivation, and/or in connection with serum free production of biopharmaceuticals. Viability described in the art is limited to 40% of cells after a 7-day batch cultivation (Goswami et al., 1999). Therefore, host cells characterized accordingly are within the meaning of the present invention.

"Cell viability" or "cell survival" is the ability of a target cell to continue to remain alive and functional, and include the protection of the cell from cell death due to inhibition or delay of apoptosis or natural cell death. Ways of measuring cell viability or survivability are well known in the art. For example, cell viability can be determined by phase contrast microscopy, fluorescence microscopy or flow cytometry using non-cell permeable dyes such as trypan blue, propidium iodide or a combination of propidium iodide/acridine orange. Those methods are exemplary described in Current Protocols in Cytometry, John Wiley & Sons, Inc., updated, incorporated by reference.

Therefore, the present invention also concerns methods for inhibiting or delaying cell death in a host cell, comprising cultivating host cells of this invention, e.g. host cells as described above, under condition where at least the anti-apoptosis gene is expressed in such that cell death is inhibited or delayed in said host cells. In a preferred embodiment, cell death of said cells is caused by programmed cell death, more preferably by apoptosis.

The present invention also provides methods of generating mammalian host cells showing an enhanced expression level of an anti-apoptosis gene comprising, (*i*.) introducing into a mammalian cell population nucleic acid sequences that encode for an anti-apoptosis gene, a selectable amplifiable marker gene, and optionally at least one gene of interest, wherein said genes are operatively linked to at least one regulatory sequence allowing for expression of said genes, (*ii*.) cultivating said cell population under conditions where at least said selectable amplifiable marker gene and said anti-apoptosis gene are expressed, and which are favorable for obtaining multiple copies at least of the anti-apoptosis gene, and (*iii*.) selecting cells from the cell population that incorporate multiple copies at least of the anti-apoptosis gene.

Host cells genetically modified by any method according to this invention show an increased expression of an anti-apoptosis protein compared to non-transfected as well as non-amplified parental cells. A "parental cell" means a cell that does not show enhanced expression of the anti-apoptosis gene, and is generally grown under the same or substantially the same conditions as the genetically modified cell according to the present invention, e.g. modified by introducing and amplifying the nucleic acid sequences that encode for the anti-apoptosis gene(s), a selectable amplifiable marker gene, and optionally at least one gene of interest. "Increased expression" of the anti-apoptosis protein means for example an over-expression, that is at least 30-fold increased, preferably at least 50-fold increased, even more preferably at least 100-fold increased compared to the expression-level of that protein in host cells which are not modified according to any method of this invention, e.g modified by introducing and amplifying the nucleic acid sequences that encode for the anti-apoptosis gene(s), a selectable amplifiable marker gene, and optionally at least one gene of interest.

Therefore, this invention also provides host cells, that are genetically modified by any method described herein and showing an over-expression of an anti-apoptosis gene that is at least about 30-fold increased compared to the expression-level of said gene in host cells which are not modified according to any method of this invention, are provided by this invention. In a further embodiment, the present invention also concerns to host cells showing a level of over-expression which is at least 50-fold increased, compared to cells not modified in the meaning of this invention. In another embodiment host cells are provided, showing a level of over-expression that is at least about 100-fold increased compared to the expression-level of cells which are not modified in any way disclosed by this invention. Expression-level achievable by host cells generated by a method of the present invention are described examplary in Figure 7. Expression levels of an anti-apoptosis gene can be further increased by applying the principles of this invention on the modification procedure of host cells, e.g. by further increasing the copy number of the anti-apoptosis gene. Also conceivable is the introduction of multiple copies of one or more anti-apoptosis genes into a host cell.

"Host cells" or "target cells" in the meaning of the present invention are hamster cells, preferably BHK21, BHK TK⁻, CHO, CHO-K1, CHO-DUKX, CHO-DUKX B1, and CHO-DG44 cells or the derivatives/progenies of any of such cell line. Particularly preferred are CHO-DG44, CHO-DUKX, CHO-K1 and BHK21, and even more preferred CHO-DG44 and CHO-DUKX cells. In a further embodiment of the present invention host cells also mean murine myeloma cells, preferably NS0 and Sp2/0 cells or the derivatives/progenies of any of such cell line. Examples of murine and hamster cells which can be used in the meaning of this invention are also summarized in Table 1. However, derivatives/progenies of those cells, other mammalian cells, including but not limited to human, mice, rat, monkey, and rodent cell lines, or eukaryotic cells, including but not limited to yeast, insect and plant cells, can also be used in the meaning of this invention, particularly for the production of biopharmaceutical proteins.

**TABLE 1:**

| **Hamster and murine production cell lines** | |
|---|---|
| **Cell line** | **Order Number** |
| NS0 | ECACC No. 85110503 |
| Sp2/0-Ag14 | ATCC CRL-1581 |
| BHK21 | ATCC CCL-10 |
| BHK TK⁻ | ECACC No. 85011423 |
| HaK | ATCC CCL-15 |
| 2254-62.2 (BHK-21 derivative) | ATCC CRL-8544 |
| CHO | ECACC No. 8505302 |
| CHO-K1 | ATCC CCL-61 |
| CHO-DUKX (= CHO duk⁻, CHO/dhfr⁻) | ATCC CRL-9096 |
| CHO-DUKX B 1 | ATCC CRL-9010 |
| CHO-DG44 | Urlaub et al., Cell 33[2], 405-412, 1983 |
| CHO Pro-5 | ATCC CRL-1781 |
| V79 | ATCC CCC-93 |
| B14AF28-G3 | ATCC CCL-14 |
| CHL | ECACC No. 87111906 |

Host cells are most preferred, when being established, adapted, and completely cultivated under serum free conditions, and optionally in media which are free of any protein/peptide of animal origin. Commercially available media such as Ham's F12 (Sigma, Deisenhofen, Germany), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, CA), CHO-S-Invtirogen), serum-free CHO Medium (Sigma), and protein-free CHO Medium (Sigma) are exemplary appropriate nutrient solutions. Any of the media may be supplemented as necessary with a variety of compounds examples of which are hormones and/or other growth factors (such as insulin, transferrin, epidermal growth factor, insulin like growth factor), salts (such as sodium chloride, calcium, magnesium, phosphate), buffers (such as HEPES), nucleosides (such as adenosine, thymidine), glutamine, glucose or other equivalent energy sources, antibiotics, trace elements. Any other necessary supplements may also be included at appropriate concentrations that would be known to those skilled in the art. In the present invention the use of serum-free medium is preferred, but media supplemented with a suitable amount of serum can also be used for the cultivation of host cells. For the growth and selection of genetically modified cells expressing the selectable gene a suitable selection agent is added to the culture medium.

The present invention also concerns high-level expression of an anti-apoptosis gene of the bcl-2 superfamily in order to improve the viability of the host cells, preferably of production cells as described above. Genes encoding for the bcl-2 superfamily are therefore suitable to generate host cells according to this invention. Thus host cells genetically modified by introducing nucleic acid sequences that encode for a gene of the bcl-2 superfamily, a selectable amplifiable marker gene, and at least one gene of interest are within the meaning of the present invention. In Table 2 examples of members belonging to the bcl-2 superfamily, that inhibit or delay programmed cell death or apoptosis, are listed. Most proteins of this family of mammalian, C. elegans or viral origin have two to four regions with extensive amino acid sequence similarity with BCL-2 (BCL-2 homology regions BH1-BH4), the prototypical inhibitor of apoptosis. All these are suitable candidates of anti-apoptosis genes/proteins to carry out the present invention.

In a further embodiment of this invention cells are preferred, wherein survival is prolonged by introducing nucleic acid sequences encoding for BCL-xL, BCL-2, BCL-w, BFL-1, A1, MCL-1, BOO, BRAG-1, NR-13, CDN-1, CDN-2, CDN-3, BHRF-1, LMW5-HL or CED-9. In a more preferred embodiment, the nucleic acid sequence encoding the anti-apoptosis gene of the present invention is a DNA molecule from a vertebrate species. A preferred vertebrate is a mammal. More preferably, a nucleic acid of the present invention encodes polypeptides designated BCL-xL or BCL-2. The BCL-xL encoding gene is the most preferred. Also preferred is a nucleic acid having the sequence of SEQ ID NO:1 (identical with GenBank Accession Number Z23115) or SEQ ID NO:2 (identical with GenBank Accession Number M13995). Most preferred is a sequence of SEQ ID NO:1. Any homologs of any of these genes encoded from other vertebrate species are also suitable to carry out the present invention. Moreover, also preferred are nucleotide sequences encoding for any of the anti-apoptosis genes of Table 2, particularly having the sequence given by any of the Genbank Accession Numbers cited in Table 2. Examples for anti-apoptosis acting gene are also described in US patents No. 6,303,331, No. 5,834,309 and No. 5,646,008 as well as in WO 95/006342, which are herein incorporated by reference. Reference is also made to Boise et al., Cell 74, 597 - 608, 1993. In WO 95/006342 isolation of the BCL-xL encoding sequence is exemplary described.

A nucleic acid sequence encoding for an anti-apoptosis protein is intended to include any nucleic acid sequence that will be transcribed and translated into an anti-apoptosis protein either *in vitro* or upon introduction of the encoding sequence into a target cell. The anti-apoptosis protein encoding sequences can be native (wild-type) genes as well as naturally occurring (by spontaneous mutation), or recombinantly engineered mutants and variants, truncated versions and fragments, functional equivalents, derivatives, homologs and fusions of the naturally occurring or wild-type proteins as long as the biological functional activity, meaning the anti-apoptotic function, of the encoded polypeptide is maintained and not substantially altered. A preferred encoded polypeptide has at least 50%, more preferred at least 80% and even more preferred at least 100% or more of functional biological activity compared to the corresponding wild-type anti-apoptosis protein. "Wild-type protein" means, a complete, non truncated, non modified, naturally occurring allele of the encoding polypeptide.

The "functional biological activity" of an anti-apoptosis polypeptide can be determined by quantitative apoptosis assays on transfected cells expressing the particular polypeptide such as, for example, TUNEL assay, Annexin V assay, acridine orange/ethidum bromide staining or propidium iodide/acridine orange staining using fluorescence microscopy or flow cytometry analysis or other assays. Those assays are well known in the art and for example described in Current Protocols in Cytometry, John Wiley & Sons, Inc., updated).

The modified nucleic acid sequences can be prepared using standard techniques well known to one of skill in the art, e.g. site-specific mutagenesis or polymerase chain reaction mediated mutagenesis. In the present invention, a particularly preferred bcl-xL nucleic acid sequence is the isolated polynucleotide of SEQ ID NO:1 (identical with GenBank Accession Number Z23115). The invention further includes functionally equivalent nucleic acid sequences to the sequence of GenBank Accession No. Z23115 and other BCL-xL encoding sequences. In one preferred embodiment, the nucleic acid sequence encoding a BCL-xL protein is of human origin, in a more preferred embodiment it is of hamster origin. However, nucleic acid sequences encoding BCL-xL from other species are encompassed in the meaning of this invention. BCL-xL protein is encoded by a bcl-x gene. This gene produces two different RNA molecules one of which codes for BCL-xL (long form) and one of which codes for BCL-xS (short form). The BCL-xS lacks a section of 63 amino acids found in the BCL-xL. BCL-xS has been shown to favor apoptosis, and therefore it is preferable to use a cDNA for expression of the BCL-xL rather than a genomic fragment. In another preferred embodiment a BCL-xL mutant, or a BCL-2 mutant, with improved anti-apoptosis properties is encompassed, e.g. by deleting a non-conserved region between the BH3 and BH4 conserved regions and thus increasing the protein stability of the mutant protein variants (Chang et al., 1997; Figueroa et al., 2001).

**TABLE 2:**

| **Anti-apoptosis genes of the bcl-2 superfamily** | | | |
|---|---|---|---|
| **Polynucleotide** | **GenBank Accession No.** | **Polynucleotide** | **GenBank Accession No.** |
| bcl-xL (human) | Z23115 | nr-13 (chicken) | AF120211 |
| bcl-xL (mouse) | L35049 | | |
| bcl-xL (rat) | U34963 | | |
| bcl-xL (chicken) | Z23110 | | |
| bcl-2 (human) | M13995 | bhfr-1 | AF120456 |
| bcl-2 (mouse) | M16506 | (herpesvirus) | |
| bcl-2 (rat) | L14680 | bhfr-1 (Epstein- | A22899 |
| bcl-2 (chicken) | Z11961 | Barr virus) | |
| bcl-2 (hamster) | AJ271720 | | |
| bcl-w (human) | U59747 | lmw5-HL (African | L09548 |
| bcl-w (mouse) | U59746 | swine fever virus) | |
| bcl-w (rat) | AF096291 | | |
| bfl-1 (human) | U27467 | cdn-1 (human) | AAQ95492 |
| A1 (mouse) | U23774 | | (NAGENESEQ) |
| mcl-1 (human) | AF147742 | cdn-2 (human) | AAQ95493 |
| mcl-1 (mouse) | U35623 | | (NAGENESEQ) |
| mcl-1 (rat) | AF115380 | | |
| mcl-1 (chicken) | AF120210 | | |
| boo (mouse) | AF102501 | cdn-3 (human) | AAQ95494 (NAGENESEQ) |
| brag-1 (human) | S82185 | ced-9 (C. elegans) | L26545 |

A "nucleic acid sequence" as used herein refers to an oligonucleotide, nucleotide or polynucleotide and fragments and portions thereof and to DNA or RNA of genomic or synthetic origin, which may be single or double stranded and represent the sense or antisense strand. The polynucleotides of the invention include nucleic acid regions wherein one or more codons have been replaced by their synonyms. Additionally included are polynucleotides which have a coding sequence which is a naturally occurring allelic variant of a coding sequence, for example, a variant of the coding sequence of SEQ ID NO:1, or SEQ ID NO:2. As known in the art, an allelic variant is an alternate form of a polynucleotide, which may have a substitution, deletion or addition of one or more nucleotides which does not substantially alter the function of the encoded polypeptide. The function of a polypeptide is in general determined by the functional biological activity of said encoding polypeptide. Therefore, not substantially alter the function of the encoded polypeptides means, that the level of functional biological activity of the encoded polypeptide is at least 50%, more preferred at least 80% and even more preferred at least 100% or more compared to the corresponding wild-type encoded polypeptide.

The term "encoding" refers to the inherent property of specific sequences of nucleotides in a nucleic acid, such as a gene in chromosome or a mRNA, to serve as templates for synthesis of other polymers and macromolecules in biological processes having a defined sequence of nucleotides (i.e. rRNA, tRNA, other RNA molecules) or amino acids and the biological properties resulting therefrom. Thus a gene encodes a protein, if transcription and translation of mRNA produced by that gene produces the protein in a cell or other biological system. Both the coding strand, the nucleotide sequence of which is identical to the mRNA sequence and is usually provided in sequence listings, and non-coding strand, used as the template for the transcription, of a gene or cDNA can be referred to as encoding the protein or other product of that gene or cDNA. A nucleic acid that encodes a protein includes any nucleic acids that have different nucleotide sequences but encode the same amino acid sequence of the protein due to the degeneracy of the genetic code. Nucleic acids and nucleotide sequences that encode proteins may include introns.

The term "cDNA" in the context of this invention refers to deoxyribonucleic acids produced by reverse transcription and typically second-strand synthesis of mRNA or other RNA produced by a gene. If double-stranded, a cDNA molecule has both a coding or sense and a non-coding or antisense strand.

In general as used herein upper case letters (e.g. BCL-xL) indicate polypeptides (e.g. products of gene expression) and lower case letters (e.g. bcl-xL) indicates polynucleotides (e.g. genes). Additionally, throughout this specification, the singular form "a", "an" and "the" include plural references unless the context clearly dictates otherwise.

The term "polypeptide" is used interchangeably with amino acid residue sequences or protein and refers to polymers of amino acids of any length. These terms also include proteins that are post-translationally modified through reactions that include, but are not limited to, glycosylation, acetylation, phosphorylation or protein processing. Modifications and changes, for example fusions to other proteins, amino acid sequence substitutions, deletions or insertions, can be made in the structure of a polypeptide while the molecule maintains its biological functional activity. For example certain amino acid sequence substitutions can be made in a polypeptide or its underlying nucleic acid coding sequence and a protein can be obtained with like properties. As mentioned above a preferred protein is the BCL-xL protein sequence encoded by nucleic acid sequence of SEQ ID NO:1. Amino acid substitutions that provide functionally equivalent BCL-xL polypeptides by use of the hydrophathic index of amino acids (Kyte et al., J. Mol. Biol. 157: 105-132, 1982) can be prepared by performing site-specific mutagenesis or polymerase chain reaction mediated mutagenesis on its underlying nucleic acid sequence.

As mentioned above, the present invention concerns genetically modified host cells and also methods for generating such host cells. In one embodiment, the host cells comprise heterologous introduced polynucleotide sequences that encode an anti-apoptosis gene, a selectable amplifiable marker gene and optionally at least one gene of interest. The "selectable amplifiable marker gene" has the properties of a selectable marker gene, but additionally allows amplification (i.e., generating additional copies of the gene which survive in intra-chromosomal or extra-chromosomal form) of the gene itself and also of genes adjacent to that selectable amplifiable gene when cells are cultured under appropriate conditions. The present invention particularly provides host cells, comprising not only one copy of an introduced anti-apoptosis gene but comprising multiple copies of said anti-apoptosis gene, e.g. more than 5, 10, 20, 50 or 100 copies of the introduced anti-apoptosis gene.

Such host cells are obtainable, for example, by the method comprising (*i*.) introducing into a mammalian cell population nucleic acid sequences that encode for an anti-apoptosis gene, a selectable amplifiable marker gene, and optionally at least one gene of interest, wherein said genes are operatively linked to at least one regulatory sequence allowing for expression of said genes, (*ii*.) cultivating said cell population under conditions where at least said selectable amplifiable marker gene and said anti-apoptosis gene are expressed, and which are favorable for obtaining multiple copies at least of the anti-apoptosis gene, and (*iii*.) selecting cells from the cell population that incorporate multiple copies at least of the anti-apoptotic gene. Host cells generated in such a manner comprise for example at least 5 copies of the incorporated heterologous anti-apoptosis gene. In a further embodiment host cells comprise for example at least 10 copies of the incorporated heterologous anti-apoptosis gene. In another embodiment host cells are obtainable comprising at least 50 copies of the introduced heterologous anti-apoptosis gene. In further embodiment host cells comprise for example at least 100 copies of a incorporated heterologous anti-apoptotic gene. Appropriate anti-apoptosis genes are those mentioned above, in particular those listed in Table 2, e.g. those encoding for BCL-xL.

In general, host cells are preferred by the invention comprising the anti-apoptosis gene in copy numbers allowing its expression at levels which are sufficiently high to enhance survival of the cell population by inhibiting or delaying programmed cell death. More preferred are host cells not only comprising the anti-apoptosis gene in copy numbers which allow to improve cell survival but also showing high level of expression of the gene of interest, also encoded by the genetically modified host cells. Methods to find out the highest-tolerable expression level for an anti-apoptosis gene having the maximum effect on reduction of apoptosis rate without leading to dramatically decreased cell growth rates or genetic instabilities and without a negative impact on the productivity with regard to the protein of interest are well known for a skilled person in the art. They include but are not limited to, for example, generating growth curves, monitoring the viability of cells by meastuing exclusion of non-permeable dyes by microscopy or flow cytometry, quantifying apoptosis by TUNEL or Annexin V assays, determination of product titers by ELISA and evaluation of the genetic stability of the transfected genes over several passages by Southern and dot blot analysis or quantitative PCR.

In this invention, for example, a sufficiently enhanced cell survival is given, if the expression level of the anti-apopotsis gene, e.g. the bcl-xL gene, reaches intracellular survival-effective concentrations of the encoding polypeptide compared to the parental, non-amplified cell. A sufficient enhanced cell survival is provided for example, when the level for bcl-xL gene expression, or for any other anti-apoptosis gene results in a cell population having at least an increase in survival of at least about 20% after a 6-, 7-, 8-, or 9-day cultivation compared to the parental cell population. More preferred is an increase in survival of at least about 30% and even more preferred an increase in survival of at least about 50% after a 6-, 7-, 8-, or 9-day cultivation compared to the parental cell population. Sufficient cell survival is also given, when at least about 50% of the cultivated cells are viable after a cultivation period of 9 days. If the anti-apoptosis gene encodes for BCl-xL, sufficient cell survival is also given, when at least about 60% of the cell are viable after 9-day cultivation. In another embodiment a sufficient enhanced cell survival is reached, when at least about 60% of the cell population is viable after 8 days. In a further embodiment, a sufficient enhanced cell survival is achieved, when at least about 75% of the cell population is viable after 7 days, furthermore if at least about 85% of the cells are viable after 6 days.

The "selectable amplifiable marker gene" usually encodes an enzyme which is required for growth of eukaryotic cells under those conditions. For example, the selectable amplifiable marker gene may encode DHFR which gene is amplified when a host cell transfected therewith is grown in the presence of the selective agent, methotrexate (MTX). The non-limited exemplary selectable genes in Table 3 are also amplifiable marker genes, which can be used to carry out the present invention. For a review of the selectable amplifiable marker genes listed in Table 3, see Kaufman, Methods in Enzymology, 185:537-566 (1990), incorporated by reference. Accordingly, host cells genetically modified according to any method described herein are encompassed by this invention, wherein the selectable amplifiable marker gene encodes for a polypeptide having the function of dihydrofolate reductase (DHFR), glutamine synthetase, CAD, adenosine deaminase, adenylate deaminase, UMP synthetase, IMP 5'-dehydrogenase, xanthine guanine phosphoribosyl transferase, HGPRTase, thymidine kinase, thymidylate synthetase, P glycoprotein 170, ribonucleotide reductase, asparagine synthetase, arginosuccinate synthetase, ornithine decarboxylase, HMG CoA reductase, acetylglucosaminyl transferase, threonyl-tRNA synthetase or Na⁺K⁺-ATPase.

A preferred selectable amplifiable marker gene is the gene encoding dihydrofolate reductase (DHFR) which is necessary for the biosynthesis of purines. Cells lacking the DHFR gene will not grow on medium lacking purines. The DHFR gene is therefore useful as a dominant selectable marker to select and amplify genes in such cells growing in medium lacking purines.

The selection agent used in conjunction with a DHFR gene is methotrexate (MTX). The present invention therefore includes a method of generating a mammalian host cell comprising (*i*.) introducing into a mammalian host cell population an anti-apoptosis gene, a DHFR encoding gene, and (*ii*.) amplifying the anti-apoptosis gene in the presence of methotrexate. Accordingly, transfected cells are cultivable in a hypoxanthine/thymidine-free medium in the absence of serum and increasing amounts of MTX, starting with no MTX in the medium to MTX concentrations between 2 nM and 2000 nM, more typically between 2 nM and 1000 nM. The concentration of MTX is increased gradually by a factor between 2 and 10. In a preferred process, the anti-apoptosis gene additionally encodes for any one of the genes listed in Table 2. In a more preferred method the anti-apoptosis gene encodes for BCL-2 or BCL-xL, and in a even more preferred method for BCL-xL.

**TABLE 3:**

| **Selectable amplifiable marker genes** | | |
|---|---|---|
| **Selectable Amplifiable Marker Gene** | **Accession Number** | **Selection Agent** |
| Dihydrofolate reductase | M19869 (hamster) | Methotrexate (MTX) |
| | E00236 (mouse) | |
| Metallothionein | D10551 (hamster) | Cadmium |
| | M13003 (human) | |
| | M11794 (rat) | |
| CAD (Carbamoyl-phosphate synthetase:Aspartate transcarbamylase: Dihydroorotase) | M23652 (hamster) | N-Phosphoacetyl-L-aspartate |
| | D78586 (human) | |
| Adenosine deaminase | K02567 (human) | Xyl-A- or adenosine, 2'deoxycoformycin |
| | M10319 (mouse) | |
| AMP (adenylate) deaminase | D12775 (human) | Adenine, azaserine, coformycin |
| | J02811 (rat) | |
| UMP synthase | J03626 (human) | 6-Azauridine, pyrazofuran |
| IMP 5'dehydrogenase | J04209 (hamster) | Mycophenolic acid |
| | J04208 (human) | |
| | M33934 (mouse) | |
| Xanthine-guanine phosphoribosyltransferase | X00221 (E. coli) | Mycophenolic acid with limiting xanthine |
| Mutant HGPRTase or mutant thymidine kinase | J00060 (hamster) | Hypoxanthine, aminopterin, and thymidine (HAT) |
| | M13542, K02581 (human) | |
| | J00423, M68489(mouse) | |
| | M63983 (rat) | |
| | M36160 (herpesvirus) | |
| Thymidylate synthetase | D00596 (human) | 5-Fluorodeoxyuridine |
| | M13019 (mouse) | |
| | L12138 (rat) | |
| P-glycoprotein 170 (MDRI) | AF016535 (human) | Multiple drugs, e.g. adriamycin, vincristine, colchicine |
| | J03398 (mouse) | |
| Ribonucleotide reductase | M124223, K02927 (mouse) | Aphidicolin |
| Glutamine synthetase | AF150961 (hamster) | Methionine sulfoximine (MSX) |
| | U09114, M60803 (mouse) | |
| | M29579 (rat) | |
| Asparagine synthetase | M27838 (hamster) | β-Aspartyl hydroxamate, Albizziin, 5'Azacytidine |
| | M27396 (human) | |
| | U38940 (mouse) | |
| | U07202 (rat) | |
| Argininosuccinate synthetase | X01630 (human) | Canavanine |
| | M31690 (mouse) | |
| | M26198 (bovine) | |
| Omithine decarboxylase | M34158 (human) | α-Difluoromethylornithine |
| | J03733 (mouse) | |
| | M16982 (rat) | |
| HMG-CoA reductase | L00183, M12705 (hamster) | Compactin |
| | M11058 (human) | |
| N-Acetylglucosaminyl transferase | M55621 (human) | Tunicamycin |
| Threonyl-tRNA synthetase | M63180 (human) | Borrelidin |
| Na⁺K⁺-ATPase | J05096 (human) | Ouabain |
| | M14511 (rat) | |

Suitable host cells for using a DHFR encoding gene as a selectable amplifiable marker are mammalian cells, preferably murine myeloma or hamster cells. More preferred are CHO-DUKX (ATCC CRL-9096) and CHO-DG44 (Urlaub et al., Cell 33[2], 405 - 412, 1983) cells which are deficient in DHFR activity. To extend the DHFR amplification method to other cell types, a mutant DHFR gene that encodes a protein with reduced sensitivity to methotrexate may be used in conjunction with host cells that contain normal numbers of an endogenous wild-type DHFR gene (Simonsonet al., 1983; Wigler et al., 1980; Haberet al., 1982).

Another embodiment of this invention also provides host cells which are genetically modified by introducing nucleic acid sequences that encode for an anti-apoptosis gene, a DHFR gene, and at least one gene of interest. In a further embodiment, the anti-apoptosis gene of said host cells encodes for any of the anti-apoptosis gene listed in Table 2, preferably for the bcl-xL gene, more preferably for the bcl-xL gene having the sequence of SEQ ID NO:1. Host cells genetically modified by introducing nucleic acid sequences that encode for bcl-xL, the DHFR gene, and at least one gene of interest are most preferred by this invention and therefore subject of the present invention. However, in general host cells are within the meaning of the present invention comprising at least a heterologous anti-apoptosis gene, a selectable amplifiable marker gene and one gene of interest, wherein the anti-apoptosis gene is any gene of Table 2 and the selectable amplifiable marker gene is any of the genes listed in Table 3.

The term "selection agent" refers to a substance that interferes with the growth or survival of a host cell that is deficient in a particular selectable gene. For example, to select for the presence of an antibiotic resistance gene like APH (aminoglycoside phosphotransferase) in a transfected cell the antibiotic Geneticin (G418) is used. The selection agent can also comprise an "amplifying agent" which is defined for purposes herein as an agent for amplifying copies of the amplifiable gene if the selectable marker gene relied on is an amplifiable selectable marker. For example, MTX is a selection agent useful for the amplification of the DHFR gene. Examplary amplifying selection agents are but not limited to those listed in Table 3.

The present invention is suitable to generate host cells for the production of biopharmaceutical polypeptides/proteins. The invention is particularly suitable for the high-yield expression of a large number of different genes of interest by cells showing an enhanced cell viability and productivity. "Gene of interest", "selected sequence", or "product gene" have the same meaning herein and refer to a polynucleotide sequence of any length that encodes a product of interest, also mentioned by the term "desired product". The selected sequence can be full length or a truncated gene, a fusion or tagged gene, and can be a cDNA, a genomic DNA, or a DNA fragment, preferably, a cDNA. It can be the native sequence, i.e. naturally occurring form(s), or can be mutated or otherwise modified as desired. These modifications include codon optimizations to optimize codon usage in the selected host cell, humanization or tagging. The selected sequence can encode a secreted, cytoplasmic, nuclear, membrane bound or cell surface polypeptide. The "desired product" includes proteins, polypeptides, fragments thereof, peptides, antisense RNA all of which can be expressed in the selected host cell. Desired proteins can be for example antibodies, enzymes, cytokines, lymphokines, adhesion molecules, receptors and derivatives or fragments thereof, and any other polypeptides that can serve as agonists or antagonists and/or have therapeutic or diagnostic use. Examples for a desired protein/polypeptide are also given below.

By definition any sequences or genes introduced into a host cell are called "heterologous sequences" or "heterologous genes" with respect to the host cell, even if the introduced sequence or gene is identical to an endogenous sequence or gene in the host cell. For example, a hamster bcl-xL gene, introduced into a hamster host cell, is by definition a heterologous gene.

Heterologous gene sequences can be introduced into a target cell, for example a nucleic acid sequence encoding BCL-xL, by using an "expression vector", preferably an eukaryotic, and even more preferably a mammalian expression vector. Methods used to construct vectors are well known to a person skilled in the art and described in various publications. In particular techniques for constructing suitable vectors, including a description of the functional components such as promoters, enhancers, termination and polyadenylation signals, selection markers, origins of replication, and splicing signals, are reviewed in considerable details in Sambrook et al., 1989 and references cited therein. Vectors may include but are not limited to plasmid vectors, phagemids, cosmids, articificial/mini-chromosomes, or viral vectors such as baculovirus, retrovirus, adenovirus, adeno-associated virus, herpes simplex virus, retroviruses, bacteriophages. The eukaryotic expression vectors will typically contain also prokaryotic sequences that facilitate the propagation of the vector in bacteria such as an origin of replication and antibiotic resistance genes for selection in bacteria. A variety of eukaryotic expression vectors, containing a cloning site into which a polynucleotide can be operatively linked, are well known in the art and some are commercially available from companies such as Stratagene, La Jolla, CA; Invitrogen, Carlsbad, CA; Promega, Madison, WI or BD Biosciences Clontech, Palo Alto, CA.

In a preferred embodiment the expression vector comprises at least one nucleic acid sequence which is a regulatory sequence necessary for transcription and translation of nucleotide sequences that encode for a peptide/polypeptide. In a more specific embodiment, the expression vector comprises at least one regulatory sequence allowing the transcription and translation (expression) of the nucleotide sequences that encode for the anti-apoptosis gene, the selectable amplifiable maker gene, and a gene of interest.

"Regulatory sequences" include promoters, enhancers, termination and polyadenylation signals, and other expression control elements. Both inducible and constitutive regulatory sequences are known in the art to function in various cell types. Transcriptionally regulatory elements normally comprise a promoter upstream of the gene sequence to be expressed, transcriptional initiation and termination sites, and a polyadenylation signal sequence. The term transcriptional initiation site refers to the nucleic acid in the construct corresponding to the first nucleic acid incorporated into the primary transcript, i.e., the mRNA precursor; the transcriptional initiation site may overlap with the promoter sequences. The term transcriptional termination site refers to a nucleotide sequence normally represented at the 3'end of a gene of interest or the stretch of sequences to be transcribed, that causes RNA polymerase to terminate transcription. The polyadenylation signal sequence or poly-A addition signal provides the signal for the cleavage at a specific site at the 3'end of eukaryotic mRNA and the post-transcriptional addition in the nucleus of a sequence of about 100- 200 adenine nucleotides (polyA tail) to the cleaved 3'end. The polyadenylation signal sequence includes the sequence AATAAA located at about 10 - 30 nucleotides upstream from the site of cleavage, plus a downstream sequence. Various polyadenylation elements are known, e.g. SV40 late and early polyA, or BGH polyA. Translational regulatory elements include a translational initiation site (AUG), stop codon and poly A signal for each individual polypeptide to be expressed. An internal ribosome entry site (IRES) is included in some constructs. IRES is defined below. In order to optimize expression it may be necessary to remove, add or alter 5' and/or 3'untranslated portions of the nucleic acid sequence to be expressed to eliminate potentially extra inappropriate alternative translation initiation codons or other sequences that may interfere with or reduce expression, either at the level of transcription or translation. Alternatively consensus ribosome binding sites can be inserted immediately 5'of the start codon to enhance expression. To produce a secreted polypeptide, the selected sequence will generally include a signal sequence encoding a leader peptide that directs the newly synthesized polypeptide to and through the ER membrane where the polypeptide can be routed for secretion. The leader peptide is often but not universally at the amino terminus of a secreted protein and is cleaved off by signal peptidases after the protein crosses the ER membrane. The selected sequence will generally, but not necessarily, include its own signal sequence. Where the native signal sequence is absent, a heterologous signal sequence can be fused to the selected sequence. Numerous signal sequences are known in the art and available from sequence databases such as GenBank and EMBL.

The expression vector can contain a single transcription unit for expression of the anti-apoptosis gene, the selectable amplifiable marker gene and optionally the gene(s) of interest, making for example use of IRES elements or intron positioning of some of the genes to operatively link all elements to the same promoter or promoter/enhancer. Alternatively, the expression vector can have one or more transcription units and the aforementioned elements can be expressed from separate transcription units with each transcription unit containing the same or different regulatory sequences. In another alternative more than one expression vector comprising one or more transcription units, each of which can be used for the expression of one or more of the elements mentioned above, can be used and introduced into a host cell by co-transfection or in sequential rounds in any order of the introduced transcription units. Any combination of elements on each vector can be chosen as long as the transcription units are sufficiently expressed. Further elements as deemed necessary may be positioned on an expression vector such as additional anti-apoptosis gene(s), gene(s) of interest or selection markers. The prerequisite for the present invention is the co-introduction of the anti-apoptosis gene and the selectable amplifiable maker gene at the same time, either in separate transcription units on one or two vectors or both in one transcription unit in a single vector, to allow for the co-amplification of the anti-apoptosis gene along with the selectable amplifiable marker gene. In a further embodiment, the anti-apoptosis gene, the selectable amplifiable maker gene and also the gene(s) of interest are being incorporated at the same time, either in separate transcription units on one, two or more vectors or in one or more transcription unit in a single vector, to allow for the co-amplification of the anti-apoptosis gene along with the selectable amplifiable marker and the gene(s) of interest.

A "promoter" refers to a polynucleotide sequence that controls transcription of a gene or sequence to which it is operatively linked. A promoter includes signals for RNA polymerase binding and transcription initiation. A promoter used will be functional in the cell type of the host cell in which expression of the selected sequence is contemplated. A large number of promoters, including constitutive, inducible and repressible promoters from a variety of different sources, are well known in the art (and identified in databases such as GenBank) and are available as or within cloned polynucleotides (from, e.g. depositories such as ATCC as well as other commercial or individual sources). With inducible promoters, the activity of the promoter increases or decreases in response to a signal. For example, the tetracycline (tet) promoter containing the tetracycline operator sequence (tetO) can be induced by a tetracycline-regulated transactivator protein (tTA). Binding of the tTA to the tetO is inhibited in the presence of tet. For other inducible promoters including jun, fos, metallothionein and heat shock promoters, see, e.g., Sambrook et al., 1989 and Gossen et al., 1994. Among the eukaryotic promoters that have been identified as strong promoters for high-level expression are the SV40 early promoter, adenovirus major late promoter, mouse methallothionein-I promoter, Rous sarcoma virus long terminal repeat and human cytomegalovirus immediate early promoter (CMV). Other heterologous mammalian promoters include, e.g., actin promoter, immunoglobulin promoter, heat-shock promoters. The aforementioned promoters are well known in the art.

An "enhancer", as used herein, refers to a polynucleotide sequence that acts on a promoter to enhance transcription of a gene or coding sequence to which it is operatively linked. Unlike promoters, enhancers are relatively orientation and position independent and have been found 5'or 3'to the transcription unit, within an intron as well as within the coding sequence itself. Therefore, enhancers may be placed upstream or downstream form the transcription initiation site or at considerable distances from the promoter, although in practice enhancers may overlap physically and functionally with promoters. A large number of enhancers from a variety of different sources are well known in the art (and identified in databases such as GenBank, e.g. SV40 enhancer, CMV enhancer, polyoma enhancer, adenovirus enhancer) and available as or within cloned polynucleotide sequences (from, e.g., depositories such as the ATCC as well as other commercial or individual sources). A number of polynucleotides comprising promoter sequences (such as the commonly used CMV promoter) also comprise enhancer sequences. For example, all of the strong promoters listed above also contain strong enhancers.

A "transcription unit" defines a region within a construct that contains one or more genes to be transcribed, wherein the genes contained within the segment are operatively linked to each other and transcribed from a single promoter, and as result, the different genes are at least transcriptionally linked. More than one protein or product can be transcribed and expressed from each transcription unit. Each transcription unit will comprise the regulatory elements necessary for the transcription and translation of any of the selected sequence that are contained within the unit.

"Operatively linked" means that two or more nucleic acid sequences or sequence elements are positioned in a way that permits them to function in their intended manner. For example, a promoter and/or enhancer is operatively linked to a coding sequence if it acts in cis to control or modulate the transcription of the linked sequence. Generally, but not necessarily, the DNA sequences that are operatively linked are contiguous and, where necessary to join two protein coding regions or in the case of a secretory leader, contiguous and in reading frame. However, although an operatively linked promoter is generally located upstream of the coding sequence, it is not necessarily contiguous with it. Enhancers do not have to be contiguous as long as they increase the transcription of the coding sequence. For this they can be located upstream or downstream of the coding sequence and even at some distance. A polyadenylation site is operatively linked to a coding sequence if it is located at the 3'end of the coding sequence in a way that transcription proceeds through the coding sequence into the polyadenylation signal. Linking is accomplished by recombinant methods known in the art, e.g. using PCR methodology, by ligation at suitable restrictions sites or by annealing. Synthetic oligonucleotide linkers or adaptors can be used in accord with conventional practice if suitable restriction sites are not present.

The term "expression" as used herein refers to transcription and/or translation of a heterologous nucleic acid sequence within a host cell. The level of expression of a desired product in a host cell may be determined on the basis of either the amount of corresponding mRNA that is present in the cell, or the amount of the desired polypeptide encoded by the selected sequence. For example, mRNA transcribed from a selected sequence can be quantitated by Northern blot hybridization, ribonuclease RNA protection, in situ hybridization to cellular RNA or by PCR (see Sambrook et al., 1989; Ausubel et al., 1987 updated). Proteins encoded by a selected sequence can be quantitated by various methods, e.g. by ELISA, by Western blotting, by radioimmunoassays, by immunoprecipitation, by assaying for the biological activity of the protein, or by immunostaining of the protein followed by FACS analysis (see Sambrook et al., 1989; Ausubel et al., 1987 updated).

As used herein the terms "enhanced expression", "over-expression" or "high-level expression" refer to sustained and sufficiently high expression of a heterologous selected sequence, in the present invention preferentially an anti-apoptosis gene, e.g. bcl-xL or bcl-2, and/or a gene of interest, introduced into a host cell. Enhanced expression can be achieved by different means. In the present invention expression of an anti-apoptosis gene such as bcl-xL or any other gene listed in Table 2, together with a selectable amplifiable marker gene provides a more efficient method of selecting for and identifying host cells expressing a heterologous gene at high levels. The selectable amplifiable marker not only allows selection of stable transfected host cell lines but allows gene amplification of the heterologous gene of interest. The additional copies of the nucleic acid sequences may be integrated into the cell's genome or an extra artificial chromosome/mini-chromosome or may be located episomally. This approach can be combined with a fluorescence activated cell sorting (FACS)-supported selection for recombinant host cells which have co-amplified the anti-apoptosis gene by using as additional selection marker for example a fluorescent protein (e.g. GFP) or a cell surface marker. Other methods for achieving enhanced expression (either on its own or in combination with the possibilities mentioned above) may include but are not limited to use of (artificial) transcription factors or treatment of cells with natural or synthetic agents to up-regulate endogenous or heterologous gene expression, improvement of either the stability of the mRNA encoding for BCL-xL or of the protein itself (e.g. deletion of protease-susceptible regions not relevant for the biological function of the protein), increasing the translation of the mRNA, or increasing gene dosage by use of episomal plasmids (based on viral sequences for replication origins, such as SV40, polyoma, adenovirus, EBV or BPV), amplification-promoting sequences (Hemann et al., 1994), or in vitro amplification systems based on DNA-concatemers (Monaco et al., 1996). A person skilled in the art will be able to identify and quantify increased copy numbers of, for example, the amplified heterologous anti-apoptosis gene by methods of the art, e.g., by Southern blot analysis or quantitative PCR methods. It is within the knowledge of a person skilled in the art how to choose the most suitable controls and to perform such assays. One example of such a method is given by the Examples described herein.

By the methods described above, host cells can be generated in such that they comprise multiple copies at least of the introduced anti-apoptosis gene, e.g. at least 5, 10, 20, 50, or 100 copies of the introduced anti-apoptosis gene.

An "internal ribosome entry site" or "IRES" describes a sequence which functionally promotes translation initiation independent from the gene 5'of the IRES and allows two cistrons (open reading frames) to be translated from a single transcript in an animal cell. The IRES provides an independent ribosome entry site for translation of the open reading frame immediately downstream of it. Unlike bacterial mRNA which can be polycistronic, i.e., encode several different polypeptides that are translated sequentially from the mRNAs, most mRNAs of animal cells are monocistronic and code for the synthesis of only one protein. With a polycistronic transcript in a eukaryotic cell, translation would initiate from the 5'most translation initiation site, terminate at the first stop codon, and the transcript would be released from the ribosome, resulting in the translation of only the first encoded polypeptide in the mRNA. In a eukaryotic cell, a polycistronic transcript having an IRES operably linked to the second or subsequent open reading frame in the transcript allows the sequential translation of that downstream open reading frame to produce the two or more polypeptides encoded by the same transcript. The IRES can be of varying length and from various sources, e.g. encephalomyocarditis virus (EMCV) or picomavirus. Various IRES sequences and their use in vector construction has been previously described (Pelletier et la., 1988; Jang et al., 1989; Davies et al., 1992; Adam et al., 1991; Morgan et al., 1992; Sugimoto et al., 1994; Ramesh et al., 1996; Mosser et al., 1997). The downstream coding sequence isoperatively linked to the 3'end of the IRES at any distance that will not negatively affect the expression of the downstream gene. The optimum or permissible distance between the IRES and the start of the downstream gene can be readily determined by varying the distance and measuring expression as a function of the distance.

The term "intron" as used herein, refers to a non-coding nucleic acid sequence of varying length, normally present within many eukaryotic genes, which is removed from a newly transcribed mRNA precursor by the process of splicing for which highly conserved sequences at or near either end of the intron are necessary. In general, the process of splicing requires that the 5' and 3'ends of the intron be correctly cleaved and the resulting ends of the mRNA be accurately joined, such that a mature mRNA having the proper reading frame for protein synthesis is produced. Many splice donor and splice acceptors sites, meaning the sequences immediately surrounding the exon-intron- and intron-exon-boundaries, have been characterized and Ohshima et al., 1987 provides a review of those.

"Transfection" of eukaryotic host cells with a polynucleotide or expression vector, resulting in genetically modified cells or transgenic cells, can be performed by any method well known in the art and described, e.g., in Sambrook et al., 1989 or Ausubel et al., 1987 (updated). Transfection methods inlcude but are not limited to liposome-mediated transfection, calcium phosphate co-precipitation, electroporation, polycation (such as DEAE-dextran)-mediated transfection, protoplast fusion, viral infections and microinjection. Preferably, the transfection is a stable transfection. The transfection method that provides optimal transfection frequency and expression of the heterologous genes in the particular host cell line and type is favored. Suitable methods can be determined by routine procedures. For stable transfectants the constructs are either integrated into the host cell's genome or an artificial chromosome/mini-chromosome or located episomally so as to be stably maintained within the host cell.

A "selectable marker gene" is a gene that only allows cells carrying the gene to be specifically selected for or against in the presence of a corresponding selection agent. By way of illustration, an antibiotic resistance gene can be used as a positive selectable marker gene that allows the host cell transformed with the gene to be positively selected for in the presence of the corresponding antibiotic; a non-transformed host cell would not be capable of growth or survival under the selection culture conditions. Selectable markers can be positive, negative or bifunctional. Positive selectable markers allow selection for cells carrying the marker by conferring resistance to a drug or compensate for a metabolic or catabolic defect in the host cell. In contrast, negative selection markers allow cells carrying the marker to be selectively eliminated. For example, using the HSV-tk gene as a marker will make the cells sensitive to agents such as acyclovir and gancyclovir. The selectable marker genes used herein, including the amplifiable selectable genes, will include recombinantly engineered mutants and variants, fragments, functional equivalents, derivatives, homologs and fusions of the native selectable marker gene so long as the encoded product retains the selectable property. Useful derivatives generally have substantial sequence similarity (at the amino acid level) in regions or domains of the selectable marker associated with the selectable property. A variety of marker genes have been described, including bifunctional (i.e. positive/negative) markers (see e.g. WO 92/08796 and WO 94/28143), incorporated by reference herein. For example, selectable genes commonly used with eukaryotic cells include the genes for aminoglycoside phosphotransferase (APH), hygromycin phosphotransferase (HYG), dihydrofolate reductase (DHFR), thymidine kinase (TK), glutamine synthetase, asparagine synthetase, and genes encoding resistance to neomycin (G418), puromycin, histidinol D, bleomycin and phleomycin.

Selection may also be made by fluorescence activated cell sorting (FACS) using for example a cell surface marker, bacterial β-galactosidase or fluorescent proteins (e.g. green fluorescent proteins (GFP) and their variants from Aequorea victoria and Renilla reniformis or other species; red fluorescent proteins, fluorescent proteins and their variants from Discosoma or other species) to select for recombinant cells.

In accordance with the teachings herein, the present invention provides also a method for the expression of an anti-apoptosis gene, a selectable amplifiable marker gene and at least one gene of interest in a host cell. Said method comprising the steps: (i.) introducing into a host cell population, preferably into a hamster host cell population, the nucleic acid sequences that encode for an anti-apoptosis gene, a selectable amplifiable marker gene, and at least one gene of interest, wherein said genes are operatively linked to at least one regulatory sequence allowing for expression of said genes, and (*ii*.) cultivating said host cell population under conditions wherein said genes are expressed. Methods, teaching to a skilled person in the art how to introduce one or more polynucleotides into a host cell are exemplary described above. Moreover, examples are also given by the invention how one or more genes can be linked with at least one regulatory sequence allowing for expression of said genes. Host cells, suitable for expressing said genes are those mentioned by the invention. Suitable candidates of an anti-apoptosis gene are listed under Table 2. Preferred is the expression of any of the sequences encoding BCL-xL or BCL-2, more preferred any sequence encoding BCL-xL. In a further preferred embodiment of that method, co-expressing host cells include any of the sequences encoding for any one of the selectable amplifiable marker listed in Table 3. Even more preferred is a method, wherein the host cells comprise a sequence encoding for a heterologous BCL-xL and also sequences encoding for any one of the selectable amplifiable marker listed in Table 3. Most preferred is a method, where the host cell comprises the sequences encoding for a heterologous BCL-xL and DHFR.

Methods provided by the present invention allows a person skilled in the art to generate new host cells, particularly for production purposes, showing enhanced cell survival attributed to an delayed or inhibited programmed cell death. Beneficial strategies have been found that dramatically increase viability and productivity of cells, constantly grown in suspension and especially in serum free-medium. Cells according to this invention are highly suitable for the production of several desired polypeptides. The genetically modified host cells described herein not only encode for the genes that are responsible for the enhanced cell survival, e.g. an anti-apoptosis gene and an amplifiable selectable marker gene, but optionally for any gene of interest encoding for a desired peptide. Accordingly, the present invention also provides to persons skilled in the art a process for producing a protein of interest in a host cell, comprising (*i*.) cultivating any host cells of this invention under conditions which are favorable for the expression of the anti-apoptosis gene and the gene(s) of interest, and (*ii*.) isolating the protein of interest from the cells and/or the cell culture supernatant. Also provided is the use of said cells for the production of at least one desired protein encoded by a gene of interest.

Desired proteins are those mentioned above. Especially, desired proteins/polypeptides are for example, but not limited to insulin, insulin-like growth factor, hGH, tPA, cytokines, such as interleukines (IL), e.g. IL-I, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (IFN) alpha, IFN beta, IFN gamma, IFN omega or IFN tau, tumor necrosisfactor (TNF), such as TNF alpha and TNF beta, TNF gamma, TRAIL; G-CSF, GM-CSF, M-CSF, MCP-1 and VEGF. Also included is the production of erythropoietin or any other hormone growth factors. The method according to the invention can also be advantageously used for production of antibodies or fragments thereof. Such fragments include e.g. Fab fragments (Fragment antigen-binding = Fab). Fab fragments consist of the variable regions of both chains which are held together by the adjacent constant region. These may be formed by protease digestion, e.g. with papain, from conventional antibodies, but similar Fab fragments may also be produced in the mean time by genetic engineering. Further antibody fragments include F(ab')2 fragments, which may be prepared by proteolytic cleaving with pepsin.

Using genetic engineering methods it is possible to produce shortened antibody fragments which consist only of the variable regions of the heavy (VH) and of the light chain (VL). These are referred to as Fv fragments (Fragment variable = fragment of the variable part). Since these Fv-fragments lack the covalent bonding of the two chains by the cysteines of the constant chains, the Fv fragments are often stabilised. It is advantageous to link the variable regions of the heavy and of the light chain by a short peptide fragment, e.g. of 10 to 30 amino acids, preferably 15 amino acids. In this way a single peptide strand is obtained consisting of VH and VL, linked by a peptide linker. An antibody protein of this kind is known as a single-chain-Fv (scFv). Examples of scFv-antibody proteins of this kind known from the prior art are described in Huston et al. (1988, PNAS 16: 5879-5883).

In recent years, various strategies have been developed for preparing scFv as a multimeric derivative. This is intended to lead, in particular, to recombinant antibodies with improved pharmacokinetic and biodistribution properties as well as with increased binding avidity. In order to achieve multimerisation of the scFv, scFv were prepared as fusion proteins with multimerisation domains. The multimerisation domains may be, e.g. the CH3 region of an IgG or *coiled coil* structure (helix structures) such as *Leucin-zipper* domains. However, there are also strategies in which the interaction between the VH/VL regions of the scFv are used for the multimerisation (e.g. dia-, tri- and pentabodies). By diabody the skilled person means a bivalent homodimeric scFv derivative. The shortening of the *Linker* in an scFv molecule to 5- 10 amino acids leads to the formation of homodimers in which an inter-chain VH/VL-superimposition takes place. Diabodies may additionally be stabilised by the incorporation of disulphide bridges. Examples of diabody-antibody proteins from the prior art can be found in Perisic et al. (1994, Structure 2: 1217-1226).

By minibody the skilled person means a bivalent, homodimeric scFv derivative. It consists of a fusion protein which contains the CH3 region of an immunoglobulin, preferably IgG, most preferably IgG1 as the dimerisation region which is connected to the scFv via a *Hinge region* (e.g. also from IgG1) and a *Linker* region. Examples of minibody-antibody proteins from the prior art can be found in Hu et al. (1996, Cancer Res. 56: 3055-61).

By triabody the skilled person means a: trivalent homotrimeric scFv derivative (Kortt et al. 1997 Protein Engineering 10: 423-433). ScFv derivatives wherein VH-VL are fused directly without a linker sequence lead to the formation of trimers.

The skilled person will also be familiar with so-called miniantibodies which have a bi-, tri- or tetravalent structure and are derived from scFv. The multimerisation is carried out by di-, tri- or tetrameric coiled coil structures (Pack et al., 1993 Biotechnology 11:, 1271-1277; Lovejoy et al. 1993 Science 259: 1288-1293; Pack et al., 1995 J. Mol. Biol. 246: 28-34).

Preferred host cells of the present invention are those described herein and which are being characterized by an enhanced cell survival. In particular, preferred are host cells genetically modified by any method according to this invention, comprising the nucleic acid sequences that encode for an anti-apoptosis gene, a selectable amplifiable marker gene and at least one gene of interest. More preferred are host cells comprising multiple copies of those genes, at least of the anti-apoptosis gene and the selectable amplifiable marker gene. Multiple copies of those genes are obtainable by methods also described herein, e.g by gene-amplification processes, and/or by providing host cells with multiple gene copies in any other way, known to person skilled in the art (e.g. introducing concatemers of the gene(s)).

Suitable anti-apoptosis genes are but not limited to those listed in Table 2. Preferred are host cells comprising multiple copies of a BCL-xL encoding gene and multiple copies of any one of the selectable amplifiable marker genes listed in Table 3. The most preferred selectable amplifiable marker gene is a DHFR encoding gene.

Host cells suitable for protein production are showing over-expression of the anti-apoptosis protein that is at least about 30-fold increased, preferably at least about 50-fold, even more preferably at least about 100-fold increased compared to the expression-level of said protein in host cells which are not modified according to any method of this invention.

Furthermore, suitable is a population of host cells showing a viability of at least about 50% when cultivated for 9 days. Furthermore, host cells are suitable and preferred by the present invention that show a viability of at least about 60% after 8 days of cultivation. Moreover, host cells are suitable in the meaning of this invention, when at least about 75% of the cell population are viable for at least 7 days. In another embodiment, a host cell population is suitable and preferred, that show a cell viability of at least about 85% after a 6-day cultivation. In the case that the anti-apoptosis encodes for BCI-xL cell viability of about 60% after 9 day cultivation, 75% after 8-day cultivation, and about 85% after 7 day cultivation are obtainable (e.g. see Figure 5). Also, host cells are particularly suitable, having at least an increase in survival of at least about 20% after a 6-, 7-, 8-, or 9-day cultivation compared to the parental cell population, which is not modified by gene-amplification methods. More preferred are those host cell populations having an increase in survival of at least about 30%, and even more preferred are host cell populations having an increase in survival of about at least about 50% after a 6-, 7-, 8-, or 9-day cultivation compared to the parental cell population.

Suitable host cells or host cell population in the meaning of the present invention includes hamster cells, preferably BHK21, BHK TK⁻, CHO, CHO-K1, CHO-DUKX, CHO-DUKX B1, and CHO-DG44 cells or the derivatives/progenies of any of such cell line. Particularly preferred are CHO-DG44, CHO-DUKX, CHO-K1 and BHK21, and even more particularly CHO-DG44 and CHO-DUKX cells. In a further embodiment of the present invention host cells also mean murine myeloma cells, preferably NS0 and Sp2/0 or the derivatives/progenies of any of such cell line. However, derivatives/progenies of those cells, other mammalian cells, including but not limited to human, mice, rat, monkey, and rodent cell lines, or eukaryotic cells, including but not limited to yeast, insect and plant cells, can also be used in the meaning of this invention for production purposes.

The selection of recombinant host cells showing increased viability and expressing high levels of a desired protein generally is a multi-step process. Selection strategy depends on whether cells are co-transfected at least by all three relevant genes (anti-apoptosis gene, selectable amplifiable marker gene(s), gene(s) of interest, and optionally additional selection marker gene(s)) in parallel, whether the gene(s) of interest will be introduced into cells already showing increased cell viability as described herein or whether the anti-apoptosis gene(s) along with a selectable amplifiable marker will be introduced into host cells expressing already a gene(s) of interest.

In the first case, transfected cells are screened or selected for the expression of any selection marker(s) co-transfected with the gene of interest, to identify cells that have incorporated the gene(s) of interest. Typically, the transfected host cells are subjected to selection for expression of the selectable marker(s) by culturing in selection medium, e.g. for about 2 weeks. Following that, cells are exposed stepwise to successively increasing amounts of the amplifying selection agent(s) to co-amplify the nucleic acid sequences encoding for at least the anti-apoptosis gene(s),the selectable amplifiable marker gene(s) and the gene(s) of interest until sufficient expression of the heterologous desired product(s) and also of the anti-apoptosis gene product(s) is obtained. Each selection step can be performed on cell pools or it can be combined with clone selection by, e.g., limited dilution. Selection can be supported by fluorescence activated cell sorting (FACS) if an appropriate marker such as GFP is used for selection.

In the second case, suitable host cells are transfected by at least the gene(s) of interest and the selectable marker gene(s), optionally a selectable amplifiable gene, which differs from the selectable amplifiable marker gene used before to generate the host cells of enhanced viability. Afterwards transfected cells are screened or selected at least for the expression of the selection marker co-transfected with the gene of interest, to identify cells that have incorporated the gene of interest. Typically, the transfected host cells are subjected to selection for expression of the selectable marker(s) by culturing in selection medium. Optionally, the selection medium also contains the selective agent which is specific for the selectable amplifiable marker used before to generate a host cell of enhanced viability. The selection step can be performed on cell pools or it can be combined with clone selection by, e.g., limited dilution. Selection can be supported by fluorescence activated cell sorting (FACS) if an appropriate marker such as GFP is used for selection. Optionally, cells can undergo further amplification steps to increase the copy number at least of the gene of interest, if another selectable amplifiable marker was used for transfection.

In the third case, recombinant host cells or production cell lines already expressing the gene(s) of interest are co-transfected with the anti-apoptosis gene(s), the selectable amplifiable marker gene(s) and optionally with additional selection marker gene(s) and/or gene(s) of interest, whereby markers different from the ones used for establishing the recombinant production cell line are used for selection. The transfected host cells are subjected to selection for expression of the newly introduced marker gene(s) by culturing in selection medium for about 2 weeks. Optionally, the selection medium contains also the selective agent which is specific for the selectable marker(s) used before to generate the recombinant production cell line. Following that, the cells are exposed stepwise to successively increasing amounts of the amplifying selection agents to co-amplify at least the anti-apoptosis gene(s) and the selectable amplifiable marker gene and optionally co-transfected gene(s) of interest until sufficient expression of at least the anti-apoptosis gene product is obtained. Each selection step can be performed on cell pools or it can be combined with clone selection by, e.g., limited dilution. Selection can be supported by fluorescence activated cell sorting (FACS), if an appropriate marker gene such as GFP is used for selection.

The protein of interest is preferably recovered from the culture medium as a secreted polypeptide, or it can be recovered from host cell lysates if expressed without a secretory signal. It is necessary to purify the protein of interest from other recombinant proteins and host cell proteins in a way that substantially homogenous preparations of the protein of interest are obtained. As a first step, cells and/or particulate cell debris are removed from the culture medium or lysate. The product of interest thereafter is purified from contaminant soluble proteins, polypeptides and nucleic acids, for example, by fractionation on immunoaffinity or ion-exchange columns, ethanol precipitation, reverse phase HPLC, Sephadex chromatography, chromatography on silica or on a cation exchange resin such as DEAE. In general, methods teaching a skilled person how to purify a protein heterologous expressed by host cells, are well known in the art. Such methods are for example described by Harris and Angal, Protein Purification Methods, in Rickwood and Hames eds., The Practical Approach Series, IRL Press (1995) or Robert Scopes, Protein Purification, Springer-Verlag (1988), both incorporated by reference.

One general subject of the present invention is to provide a person skilled in the art with host cells showing an increased cell survival. Cell survival can be increased by inhibiting or delaying programmed cell death, e.g, apoptosis, by any methods as described herein. The most sufficient way to enhance cell survival in the meaning of this invention is to provide a host cell with multiple copies of at least a heterologous introduced anti-apoptosis gene and culturing the host cells under conditions allowing high expression of those anti-apoptosis gene copies. The present invention also provides host cells, comprising at least 5 copies of a heterologous anti-apoptosis gene. Also provided are host cells, comprising at least 10 copies of a heterologous anti-apoptosis gene. Moreover, the present invention also provides host cells, comprising at least 20 copies, in another embodiment at least 50 copies, and in a further embodiment at least 100 copies of the heterologous anti-apoptosis gene(s). The present invention provides several methods to a skilled person in the art how to generate such host cells, comprising multiple copies of an heterologous anti-apoptosis gene. Those methods include but are not limited to those described above: e.g. (*i*.) stepwise amplification of at least one heterologous introduced anti-apoptosis gene driven by an selective agent, (*ii*.) increasing gene dosage of at least one heterologous introduced anti-apoptosis gene by use and introduction of episomal plasmids as described above, (*iii*.) or using in vitro amplification systems based on DNA-concatemers as described for example by Monaco et al., 1996, incorporated herein by reference.

Suitable anti-apoptosis genes are any of those listed in Table 2, particularly encoded by any of the nucleic acid sequences referred to in Table 2. Preferred is a host comprising multiple copies of a gene encoding for BCL-xL or BCL-2, more preferred for BCL-xL, and even more preferred for BCL-xL encoded by a nucleic acid having the sequence of SEQ ID NO:1. In a further embodiment the anti-apoptosis gene encoding BCL-2 or having the sequence of SEQ ID NO:2 is preferred. Also preferred are host cells comprising different anti-apoptosis genes, e.g. selected from those listed in Table 2. In a more preferred embodiment of this invention, at least one copy of the mixture encodes for a heterologous BCL-xL.

Suitable host cells are any of those described in the present invention, e.g. those mentioned in Table 1. More preferred are host cells of hamster or murine origin, e.g. murine myeloma cells. Even more preferred are CHO or BHK cells, particularly CHO-DG44, BHK21, BHK TK⁻, CHO, CHO-K1, CHO-DUKX, CHO-DUKX B1, and CHO-DG44 cells or the derivatives/progenies of any of such cell line. Most preferred are CHO-DG44, CHO-DUKX, CHO-K1 and BHK21, particularly CHO-DG44 and CHO-DUKX cells.

Host cells described above can be additionally modified by introducing at least one heterologous gene of interest such that the present invention also concerns host cells comprising multiple copies of an anti-apoptosis gene as described above, and further comprising at least one gene of interest.

The practice of the present invention will employ, unless otherwise indicated, conventional techniques of cell biology, molecular biology, cell culture, immunology and the like which are in the skill of one in the art. These techniques are fully disclosed in the current literature. See e.g. Sambrook et al., Molecular Cloning: A Laboratory Manual, 2^{nd} Ed., Cold Spring Harbor Laboratory Press, Cold Spring Harbor, N.Y. (1989); Ausubel et al., Current Protocols in Molecular Biology (1987, updated); Brown ed., Essential Molecular Biology, IRL Press (1991); Goeddel ed., Gene Expression Technology, Academic Press (1991); Bothwell et al. eds., Methods for Cloning and Analysis of Eukaryotic Genes, Bartlett Publ. (1990); Wu et al., eds., Recombinant DNA Methodology, Academic Press (1989); Kriegler, Gene Transfer and Expression, Stockton Press (1990); McPherson et al., PCR: A Practical Approach, IRL Press at Oxford University Press (1991); Gait ed., Oligonucleotide Synthesis (1984); Miller & Calos eds., Gene Transfer Vectors for Mammalian Cells (1987); Butler ed., Mammalian Cell Biotechnology (1991); Pollard et al., eds., Animal Cell Culture, Humana Press (1990); Freshney et al., eds., Culture of Animal Cells, Alan R. Liss (1987); Studzinski, ed., Cell Growth and Apoptosis, A Practical Approach, IRL Press at Oxford University Presss (1995); Melamed et al., eds., Flow Cytometry and Sorting, Wiley-Liss (1990); Current Protocols in Cytometry, John Wiley & Sons, Inc. (updated); Wirth & Hauser, Genetic Engineering of Animals Cells, in: Biotechnology Vol. 2, Pühler ed., VCH, Weinheim 663-744; the series Methods of Enzymology (Academic Press, Inc.), and Harlow et al., eds., Antibodies: A Laboratory Manual (1987).

All publications and patent applications mentioned in this specification are indicative of the level of skill of those skilled in the art to which this invention pertains. All publications and patent applications cited herein are hereby incorporated by reference in their entirety in order to more fully describe the state of the art to which this invention pertains. The invention generally described above will be more readily understood by reference to the following examples, which are hereby included merely for the purpose of illustration of certain embodiments of the present invention and are not intended to limit the invention in any way.

### EXAMPLES

### Abbreviations

- CHO:: chinese hams ter ovary
- DHFR:: dihydrofolate reductase
- ELISA:: enzyme-linked immunosorbant assay
- FACS:: fluorescence activated cell sorter
- FITC:: fluorescein isothiocyanate
- HRPO:: horseradish peroxidase
- HT:: hypoxanthine and thymidine
- IRES:: internal ribosome entry site
- MTX:: methotrexate
- PAGE:: polyacrylamide gel electrophoresis
- PBS:: phosphate buffered saline
- PI:: propidium iodide
- SEAP:: secreted alkaline phosphatase
- SDS:: sodium dodecyl sulfate
- sICAM:: soluble intracellular adhesion molecule

### Methods

### 1. Cell culture

CHO-DG44/dhfr^{-/-} (Urlaub et al., 1983), grown permanently in suspension in the serum-free medium CHO-S-SFMII (Invitrogen, Carlsbad, CA) supplemented with hypoxanthine and thymidine (Invitrogen, Carlsbad, CA), were incubated in cell culture flasks at 37°C in a humidified atmosphere containing 5% CO2. Cells were seeded at a concentration of 2x10⁵ cells/ml in fresh medium every two to three days and cell suspensions (500 µl) were analyzed for cell number and viability using a CASY1 cell counter (Schaerfe System; Germany). Viability was also confirmed by trypan blue dye exclusion. Single cell cloning was done by standard dilution technology in 96-well chambers. For the DHFR-based selection of stable transfected CHO-DG44 cells CHO-S-SFMII medium without hypoxanthine and thymidine was used.

DHFR-based gene amplification was achieved by adding 20 nM MTX (Sigma, Germany) as amplifying selection agent to the medium. For batch cultivation clones were seeded in duplicate into cell culture flasks (T25) containing 12 nil of the appropriate medium and kept for 7 or 9 days in the incubator without adding any fresh medium. When CHO-DG44 cells were cultivated in the presence of serum CHO-S-SFMII medium was supplemented with 10% fetal calf serum (Sigma, Germany). For passaging cells were trypsinized (in the presence of serum CHO-DG44 became adherent) and seeded at a concentration of 2x10⁵ cells/ml in fresh medium every two to three days.

### 2. Expression vectors

The basic vector pBID, based on the pAD-CMV vector (Werner et al., 1998), mediates constitutive expression of the heterologous genes driven by the CMV promoter/enhancer. In addition, pBID encodes the dhfr mini gene as amplifiable selectable marker (see for example EP 0 393 438). sICAM was isolated as HindIII/SalI fragment from pAD-sICAM (Werner et al., 1998) and cloned into the corresponding sites (HindIII and Sail) of pBID, resulting in pBID-sICAM. The human homologous of bcl-xL (SEQ ID NO:1, GenBank Accession Number Z23115) and bcl-2 (SEQ ID NO:3, GenBank Accession Number M13995) were cloned into the expression vectors as follows. For the construction of pBID-bcl-2, bcl-2 was at first PCR-amplified from human total RNA and subcloned into the pCR2.1-TOPO cloning vector (Invitrogen, Carlsbad, CA). From there it was excised using EcoRI and ligated into the corresponding EcoRI site of pBID. Using Klenow-DNA polymerase the filled in PmeI-excised IRES-bcl-xL fragment of pDD6 (Fussenegger et al., 1998) was cloned into the filled in XbaI site of pBID-sICAM resulting in the bicistronic expression vector pBID-sICAM-bcl-xL. This vector was the basis for the construction of pBID-bcl-xL by eliminating the sICAM-IRES element from pBID-sICAM-bcl-xL by SalI/XhoI digestion and religation. pSEAP2-Control harbors the human secreted alkaline phosphatase under control of the SV40 promoter (Clontech, Palo Alto, CA). pGL2-Control contains the firefly luciferase driven by the SV40 promoter (Promega, Madison, USA).

### 3. Transient and stable transfections

Transfections were conducted using Fugene6 reagent (Roche Diagnostics GmbH, Mannheim, Germany). Per transfection 0,6x10⁶ exponentially growing CHO-DG44 cells in 2ml HT-supplemented CHO-S-SFMII medium were seeded in a well of a 6-well chamber. A total of 4 µg plasmid-DNA and 10 µl Fugene6 reagent were used for each transfection, following the protocol of the manufacturer. Transient transfections were performed in triplicate and supernatant and cells were harvested 48 hours post transfection. For stable transfections the medium was replaced with HT-free CHO-S-SFMII medium 72 hours post transfection and the mixed cell populations were selected for two weeks prior to analysis or cloning with medium changes every 3 to 4 days. Single cell cloning was done by standard dilution technology. For amplification of the heterologous genes, integrated into the chromosomes of the host cells, single cells derived from a mixed genetically modified host cell population were seeded into 96-well chambers that contained 200 µl HT-free CHO-S-SFMII medium supplemented with 20 nM MTX. Amplified single cell clones were selected during 3 weeks and expanded into cell culture flasks.

### 4. sICAM ELISA

sICAM titers in supernatants were quantified by ELISA with standard protocols (Ausubel et al., 1994, updated) using two in house developed sICAM specific monoclonal antibodies (as described for example in US patents No. 5,284,931, 5,475,091), whereby one of the antibodies is a HRPO-conjugated antibody. Purified sICAM protein was used as a standard. Samples were analyzed using a Spectra Fluor Plus reader (TECAN, Crailsheim, Germany).

### 5. Reporter enzyme assays

The firefly luciferase reporter enzyme activity was determined with the Luciferase Assay System from Promega (Madison, WI) according to protocol. SEAP activity was measured with the Great EscAPe SEAP kit from Clontech (Palo Alto, CA) according to protocol. Samples were analyzed using a Spectra Fluor Plus reader (TECAN, Crailsheim, Germany).

### 6. Western blot analysis

The BCL-2 and BCL-xL expression of the parental host cells CHO-DG44 and the genetically modified host cells was confirmed by Western Blot analysis. About 1x10⁶ cells were extracted in 500 µl lysis buffer (1% Triton X-100, 15 mM NaCI, 10 mM Tris-HCl pH 7.5 with 50 µg/ml phenyl methanesulfonyl fluoride and 200 µl of the protease inhibitor cocktail Complete from Roche Diagnostics). The lysates were clarified by centrifugation at 13.000 xg for 10 min. Protein concentrations were determined by Bradford assay according to the manufacturer's protocol (Bio-Rad Laboratories GmbH, Munich, Germany). The extracts were then stored at -80°C until needed. Equal amounts of protein (20 µg) were subjected to 10% SDS-PAGE (Novex; Invitrogen. Carlsbad, CA) and subsequently electroblotted onto nitrocellulose membranes (Invitrogen, Carlsbad, CA). After blocking with 5% skim milk, proteins were detected using mouse monoclonal antibodies from Santa Cruz Biotechnology (Santa Cruz, CA) specific for BCL-2 or BCL-xL. Proteins were visualized with an anti-mouse peroxidase-coupled secondary antibody (Dianova GmbH, Hamburg, Germany) using ECL detection system (Amersham Biosciences, Freiburg, Germany).

### 7. Quantification of apoptosis

Fragmented DNA levels were quantified using a fluorescence-based TUNEL-assay (BD Biosciences PharMingen, San Diego, CA). 1x10⁶ cells were harvested, washed with PBS and treated with 1% paraformaldehyde solution in PBS for 15 min at 4°C. Following an additional washing step using PBS the cells were fixed in 70% ethanol and analyzed according to the manufacturer's protocol. FITC and PI emission profiles of 10.000 cells per sample were analyzed using a FACSCalibur (Becton-Dickinson).

### 8. Flow cytometry of labeled mitochondria

500.000 cells per sample were harvested, washed with PBS, and treated with 1% paraformaldehyde in PBS for 15 min at 4°C. After a second PBS wahsing step the cells were ready for the fluorescence-based staining of their mitochondria. A PBS solution containing 500 pM of the mitochondrion-selective MitoTracker Green FM dye (Molecular Probes, Eugene, OR) was prepared from a 1 mM stock solution. Cells were incubated in 200 µl staining solution for 15 min at 37°C. Subsequently, the cells were washed twice with PBS, resuspended in 400 µl PBS and subjected to flow cytometric analysis. The green fluorescence emission of 10.000 cells per sample was determined.

### Example 1: Overexpression of survival and product genes in transient and stable mode

The common cold therapeutic sICAM (soluble intercellular adhesion molecule 1) competes with the ICAM receptor for rhinovirus binding and prevents interaction of this common cold etiological agent with the ICAM receptor which is required for cell entry and subsequent infection (Bella et al., 1999; Marlin et al., 1990). For detailed assessment of transient expression of anti-apoptosis genes bcl-2 or bcl-xL on the production of sICAM equal amounts of the sICAM encoding plasmid pBID-sICAM were co-transfected in triplicate with either the pBID-bcl-2, pBID-bcl-xL or the isogenic control pBID (Figure 1) into dhfr-deficient CHO-DG44. The cells were grown permanently in suspension in the serum-free medium CHO-S-SFMII (Invitrogen, Carlsbad, CA) supplemented with hyopxanthine and thymidine. sICAM titers in supernatants were quantified 48 hours post transfection by ELISA using in house produced sICAM specific antibodies. Whereas BCL-2 expression dramatically reduced sICAM production to 10% of the control pBID, ectopic BCL-xL expression increased the yield of sICAM by 60% (Figure 2A). These data were confirmed by SEAP expression profiles (exchanging pBID-sICAM by pSEAP2-Control) demonstrating that observed production characteristics of BCL-2 and BCL-xL were of general rather than a combinatorial effect of sICAM/BCL-2 or sICAM/BCL-xL expression (Figure 2B). Also, in order to assess whether the impact of BCL-2 and BCL-xL expression on cellular production parameters is linked or limited to secreted product proteins, identical production profiling was performed using the intracellular luciferase reporter (exchanging pBID-sICAM by pGL2-Control). Luciferase production characteristics correlated with aforementioned SEAP and sICAM expression profiles confirming the positive (bcl-xL) and negative (bcl-2) impact of these apoptosis-suppression genes on the overall productivity of CHO-DG44 cells (Figure 2C). Reduced production of desired proteins following expression of BCL-2 has also been observed in COS-7 cells (ATCC CRL-1651), anchorage-dependent CHO-K1 (ATCC CCL-61) and CHO-DUKX cells (ATCC CRL-9096) adapted for growth in suspension.
Although transient transfection experiments enable highly reliable information on the metabolic engineering capacity of heterologous genes over a wide range of cell types and copy numbers, only stable mixed populations can confirm beyond doubt the observed phenotype. Therefore sICAM titer profiles of 6 mixed populations for each pBID-sICAM/pBID-bcl-2, pBID-sICAM/pBID-bcl-xL, pBID-sICAM/pBID configuration were generated. Transfected cell populations were selected for the expression of the heterologous genes by DHFR-based selection in HT-free CHO-S-SFMII medium. Figure 3 shows the absolute sICAM yield produced by those stable transfected mixed populations during a 3 day cultivation period. Each value represents an average sICAM production of all populations over five passages, whereby at each passage a sample was taken. As seen with transient transfections, BCL-2 expression has a negative impact on sICAM production with titers reduced by 50%, whereas BCL-xL expression increases the overall yield of this common cold therapeutic by 30% compared to the control populations engineered for sICAM-only expression. These correlating results obtained in transient and stable expression configurations suggest that design of anti-apoptosis engineering strategies should only be considered following careful analysis of available survival determinants.

### Example 2: Effect of dhfr-amplified gene expression of bcl-2 and bcl-xL on viability and apoptosis of stable sICAM producing cell clones

For the evaluation of the effect of anti-apoptosis engineering on the viability of transgenic CHO-DG44 cell lines in serum-free batch cultivation, the highest sICAM-producing cell populations out of 6 mixed cell populations each, generated by stable co-transfection of CHO-DG44 with either pBID-sICAM/pBID-bcl-2, pBID-sICAM/pBID-bcl-xL or pBID-sICAM/pBID vector combinations, were selected for further analysis. Two parallel single cell cloning procedures by limited cell dilution in 96-well chambers were conducted, one in HT-free CHO-S-SFMII medium (non-amplified cell clones) and one including a single round of MTX-induced DHFR-based amplification by supplementing the HT-free CHO-S-SFMII medium with 20 nM MTX (amplified cell clones). The cell clones were screened for the highest sICAM producers by ELISA and for each gene configuration the best two cell clones were selected for further analysis. In addition to pBID-sICAM these cell clones also contain one of the following constructs: (*i*.) pBID (vector-only control) in HMNI-1, HMNI-2 (not amplified) and HMNI-3, HMNI-4 (amplified), (*ii*.) pBID-bcl-2 in HMNIBC-1, HMNIBC-2 (not amplified) and HMNIBC-3, HMNIBC-4 (amplified), (*iii*.) pBID-bcl-xL in HMNIBX-1, HMNIBX-2 (not amplified) and HMNIBX-3, HMNIBX-4 (amplified).
Viability of all cell clones in batch cultivation in cell culture flasks was monitored daily for a period of one week. As seen in Figure 4 all non-amplified cell clones, including the vector-only control, exhibited similar viability profiles clearly showing the negligible effect of BCL-2 and BCL-xL on viability. At day 7 viability was down to approximately 30 - 40% in all cases. However, significant increases in cell viability could be observed during the decline phase of clones containing amplified heterologous genes. BCL-xL was clearly outperforming BCL-2 with respect to cell death protection (Figure 5). In BCL-xL expressing cell lines (HMNIBX-3/4) there were still 70% viable cells in the culture at day 9, compared to about 60% in the BCL-2 expressing cell lines (HMNIBC-3/4) and no viable cells in the transgenic control cell lines (HMNI-3/4). Correlating with an increase in viability amplified cell clones also showed a dramatic reduction in the percentage of cells which initiate apoptosis programs, assessed at days 2, 4 and 6 using a fluorescence-based TUNEL assay (Figure 6). Whereas at day 2 almost no apoptotic cells were found in all transgene configurations, a significant increase in apoptotic cell numbers was seen at day 4. The least increase up to 5% was found in BCL-xL expressing cells, followed by BCL-2 expressing cells with 10% and the transgenic control cells already with 30% apoptotic cells. At day 6 the difference between the various transfected cell lines was even more pronounced. The apoptosis-suppressing potential of BCL-xL is at least three-fold higher compared to BCL-2, where 30% of apoptotic cells compared to 10% in BCL-xL expressing cells were found at this stage. In the transgenic control cells, not expressing any heterologous anti-apoptosis gene, the number of apoptotic cells amounted already to 60%.
In order to confirm the amplification status of the anti-apoptosis genes BCL-2- and BCL-xL-directed Western blot analysis was performed. Figure 7 demonstrates only basal BCL-2 (Figure 7A) and BCL-xL (Figure 7B) expression in the transgenic control cell line HMNI-1 at levels near the detection limit and comparable to the parental CHO-DG44 cells. This indicates survival gene expression is not upregulated by DHFR-based selection as was shown to occur in an earlier report following G418-mediated selection which biased results towards enhanced survival (Tey et al., 2000a). BCL-2 and BCL-xL expression profiles could be increased at least 80- to 100-fold and 30- to 40-fold, respectively, by DHFR-based amplification compared to the transgenic control HMNI-1 and the parental CHO-DG44 (HMNIBC-3/4 and HMNIBX-3/4; Figure 12A and 12B). In the non-amplified transgenic cell lines BCL-2 and BCL-xL expression levels were increased less than 10-fold. This protein level was obviously to low for a sufficient protective anti-apoptosis effect (Figure 5). Comparable results were obtained with cells transfected with pBID-sICAM-bcl-xL, an expression vector with a dicistronic configuration. In this set-up translation of sICAM in the first cistron of the transcription unit was initiated in a classical cap-dependent manner and of bcl-xL in the second cistron in cap-independent manner driven by an IRES element derived from the encephalomyocarditis virus. The selectable amplifiable marker dhfr was contained in a separate transcription unit on the same vector.
The detailed analysis of BCL-2 and BCL-xL-mediated anti-apoptosis engineering exemplifies that high level expression of these survival determinants is required in order to exhibit any significant protective effects in CHO-DG44 cells cultivated permanently under serum-free medium conditions.

### Example 3: Determination of gene copy numbers by dot blot analysis

Copy numbers of heterologous anti-apoptosis genes bcl-2 or bcl-xL integrated in the genome of the transfected host cells CHO-DG44 are determined by dot blot analysis. For this purpose genomic DNA is isolated from the transgenic and the parental CHO-DG44 cell lines by using the commercially availabe Genomic Blood & Tissue Kit from Qiagen (Hilden, Germany) according to the protocol of the manufacturer. Various amounts of the genomic DNA, usually between 0,5 µg and 15 µg, are applied in duplicate in alkaline buffer to positively charged Hybond N+ nylon membrane (Amersham Biosciences, Freiburg, Germany) using a manifold attached to a suction device (for detailed protocol see Ausubel et al., 1994, updated). The amount of genomic DNA that should be blotted will depend on the relative abundance of the target sequence that will be subsequently sought by hybridization probing. Hybridization analysis is then carried out to determine the abundance of the bcl-2 or bcl-xL sequences in the blotted DNA preparations. Following the protocol of the Gene Images random prime labelling module (Amersham Biosciences, Freiburg, Germany) random-primed FITC-dUTP labelled bcl-2 and bcl-xL specific probes are generated, using either the bcl-2 specific 640 bp fragment obtained by EcoRI digestion of pBID-bcl-2 or the bcl-xL specific 730 bp PvuII/BclI fragment generated from digestion of pBID-bcl-xL as template in the labelling reaction. Hybridisation is performed overnight at 65°C in a hybridization oven according to the Gene Images random prime labelling module protocol. After hybridisation the filter is washed twice with 0.2x SSC/0.1% SDS at 65°C for 20 min before proceeding with the antibody development and the detection according to the Gene Images CDP-Star detection module (Amersham Biosciences). Quantification of the signals is performed using the VDS-CL Imager System (Amersham Biosciences). Signal intensities of bcl-2 or bcl-xL specific signals from the genomic DNA, isolated from the transgenic cells, are compared to a standard curve of defined molecule numbers of either the pBID-bcl-2 or the pBID-bcl-xL expression plasmid (numbers calculated on the basis of the molecular weight of the plasmids), respectively, and are used to estimate the absolute amount of target DNA within the genomic DNA samples. Hybridization signals from the genomic DNA of the parental cell line CHO-DG44, due to the endogenous bcl-2 and bcl-xL genes, are substracted from the transgenic signals. The copy numbers of the transgenic samples are then divided by the DNA content of each sample to obtain the gene copy number per cell and multiplied by 5 pg, based on 5 pg DNA content per cell. For normalization of DNA contents among samples that are being compared, part of the hamster mbh-1 gene (myc-basic motif homolog 1) is used as an internal control gene probe in a second hybridization reaction on the same filter after stripping the filter of the first probes.
By this method genetically modified host cells generated by introducing and amplifying the bcl-2 or bcl-xL gene (e.g. as described herein) can be determined, comprising at least 5, 10, 20, 50 or 100 copies of a heterologous bcl-2 or bcl-xL gene.

### Example 4: Modulation of mitochondria number by serum additives

The obvious requirement for a high BCL-2 or BCL-xL dosage in order to achieve significant survival impact in CHO-DG44 grown under serum-free conditions initiated speculations to whether the mitochondria content and therefore sensitivity to programmed cell death is dependent on serum. This hypothesis is even more convincing as serum has been found to be a key factor for apoptosis protection and because mitochondria are a major platform for cell death modulators (like BCL-2 and BCL-xL).
With a focus on determining the serum-dependence of the specific mitochondria content, CHO-DG44 cells were cultivated either in suspension in serum-free CHO-S-SFMII medium or as adherent cells in CHO-S-SFMII medium supplemented with 10% FCS. Following cultivation for two weeks under stated conditions, the cells of both cultures were profiled for their specific mitochondria content using MitoTracker Green FM, a mitochondrion-specific fluorescent dye. As MitoTracker Green FM accumulates in mitochondria in a membrane potential-independent manner it is a well-established tool for the quantification of these organelles (Metivier et al., 1998). Mitochondria-specific staining was significantly increased in cells cultivated in the absence of serum compared to cells cultivated in the presence of serum. FACS-mediated analysis quantified an up to 3-fold boost in specific mitochondria content (Figure 8). Given the clear amplification of mitochondria under serum-free conditions it is straightforward to suggest that successful anti-apoptosis engineering of serum-independent cell lines requires increased BCL-2 or BCL-xL expression levels to compensate for specific increases in apoptosis machineries associated with these organelles.

### REFERENCES CITED

Adam, M.A. et al., J *Virol* **1991,** *65*, 4985 - 4990
Al-Rubeai. M. et al., *Curr Opin Biotechnol* **1998,** 9, 152-156
Ausubel, F.M. et al., Current protocols in molecular biology. New York: Greene Publishing Associates and Wiley-Interscience. **1994** (updated)
Bella, J. et al., *J Struct Biol* **1999,** *128*, *69-74*
Boise, L.H. et al., *Cell* **1993,** *74*, 597 - 608
Bothwell et al. eds., Methods for Cloning and Analysis of Eukaryotic Genes, Bartlett Publ., **1990**
Brown ed., Essential Molecular Biology, IRL Press, 1991
Butler ed., Mammalian Cell Biotechnology, **1991**
Chang, B.S. et al., *Embo J* **1997,** *16(5)*, 968 - 977
Chung, J. D. et al., *Biotechnol Bioeng* **1998,** *57*, 164-171
Cotter, T. G. et al., *Trends Biotechnol* **1995,** *13*, 150-155
Davies, M.V. et al., *J Virol* **1992,** *66*, 1924 - 1932
deZengotita, V.M. et al., *Biotechnol Bioeng* **2000,** *69*, 566 - 576
Fassnacht, D. et al., *Cytotechnology* **1998,** *26*, 219-225
Figueroa, B. Jr. et al., *Biotechnol Bioeng* **2001,** *73*, 716-723
Follstad, B.D. et al., *Eur J Biochem* **2000,** *267*, 6534-6540
Franek, K. et al., *J FEBS Lett* **1991,** *284*, 285-287
Franek, F. et al., *Immunol Lett* **1996,** *52*, 139-144.
Freshney et al., eds., Culture of Animal Cells, Alan R. Liss, **1987**
Fussenegger, M. et al., *Nat Biotechnol* **1998,** *16*, 468-472
Fussenegger, M. et al., *Cytotechnology* **2000,** *32*, 45-61
Gait ed., Oligonucleotide Synthesis, **1984**
Goeddel ed., Gene Expression Technology, Academic Press, **1991**
Gossen, M. et al., *Curr Opi Biotech* **1994,** *5*, 516 - 520
Goswami, J. et al., *Biotechnol Bioeng* **1999,** *62*, 632-640
Green, D.R. et al., *Science* **1998,** *281*, 1309 - 1312
Haber, D.A. et al., *Somatic Cell Genetics* **1982,** *8*, 499 - 508
Harlow et al., eds., Antibodies: A Laboratory Manual, **1987**
Harris and Angal, Protein Purification: A Practical Approach, Pickwood and Hames, eds., IRL Press, **1995**
Hemann, C. et al., *DNA Cell Biol* **1994,** *13(4)*, 437 - 445
Hu et al., *Cancer Res*. **1996,** *56*, 3055 - 3061
Huston C. et al., *PNAS* **1988,** *85(16)* 5879 - 5883
Itoh, Y. et al., *Biotechnol Bioeng* **1995,** *48*, 118-122
Jang, S.K. et al., *J Virol* **1989,** *63*, 1651 - 1660
Kaufman, R.J., *Methods in Enzymology* **1990,** *185*, 537 - 566
Kortt et al., *Protein Engineering* **1997,** *10*, 423 - 433
Kriegler, Gene Transfer and Expression, Stockton Press, **1990**
Kyte, J. et al., *J Mol Biol* **1982,** *157*, 105 - 132
Laken, H.A. et al., *Curr Opin Biotechnol* **2001,** *12*, 175 - 179
Loyvejoy et al., *Science* **1993,** *259*, 1288 - 1293
Marlin, S. D. et al., *Nature* **1990,** *344*, 70-77
Mastrangelo, A. J. et al., *Trends Biotechnol* **1998,** *16*, *88-95*
Mastrangelo, A.J.et al., *Biotechnol Bioeng* **1999,** *65*, 298-305
Mastrangelo, A. J. et al., *Biotechnol Bioeng* **2000a,** *67*, 555-564
Mastrangelo, A. J. et al., *Biotechnol Bioeng* **2000b,** *67*, 644-554
McPherson et al., PCR: A Practical Approach, IRL Press at Oxford University Press, **1991**
Melamed et al., eds., Flow Cytometry and Sorting, Wiley-Liss, **1990**
Mercille, S. et al., *Biotechnol Bioeng* **1994,** *44*, 1140-1154
Mercille, S. et al., *Biotechnol Bioeng* **1999a,** *63*, 529-543
Mercille, S. et al., *Biotechnol Bioeng* **1999b,** *63*, 516-528
Metivier, D. et al., *Immunol Lett* 1998, *61*, 157-163
Miller & Calos eds., Gene Transfer Vectors for Mammalian Cells, **1987**
Monaco, L. et al., *Gene* **1996,** *180*, 145 - 150
Moore, A. et al., *Cytotechnology* **1995,** *17*, 1-11
Morgan, R.A. et al., *Nucl Acids Res* **1992,** *20*, 1293 - 1299
Mosser, D.D. et al., *BioTechniques* **1997,** *22(1)*, 150 - 156
Ohshima, Y. et al., *J Mol Biol* **1987,** *195*, 247 - 259
Pack et al., *Biotechnology* **1993,** 11, 1271 - 1277
Pack et al., *J. Mol. Biol.* **1995,** *246*, 28 - 34
Pan, G. et al., *J Biol Chem* **1998,** *273*, 5841-5845
Pelletier, J. et al., *Nature* **1988,** *334*, 320 - 325
Perisic O, et al., *Structure* **1994,** *2*, 1217 - 1226
Pollard et al., eds., Animal Cell Culture, Humana Press, **1990**
Ramesh, N. et al., *Nucl Acids Res* **1996,** *24*, 2697 - 2700
Reynolds, J.E. et al., *Exp Cell Res* **1996,** *225*, 430 - 436
Sambrook et al., Molecular Cloning: A Laboratory Manual, 2^{nd} Ed., Cold Spring Harbor Laboratory Press, Cold Spring Harbor, N.Y., 1989
Sanfeliu, A. et al., *Biotechnol Bioeng* **1999,** *64*, 46-53
Sanfeliu, A. et al., *Biotechnol Bioeng* **2000,** *70*, 421-427
Scopes, R., Protein Purification, Springer Verlag, **1988**
Simonson, C.C. et al., *Proc Natl Acad Sci USA* **1983,** *80*, 2495 *-* 2499
Simpson, N. H. et al., *Biotechnol Bioeng* **1997,** *54*, 1-16
Simpson, N.H. et al., *Biotechnol Bioeng* **1998,** *59*, 90-98
Simpson, N.H. et al., *Biotechnol Bioeng* **1999,** *64*, 174-186
Studzinski, ed., Cell Growth and Apoptosis. A Practical Approach, IRL Press at Oxford University Press **1995**
Sugimoto et al., Biotechnology **1994,** 12, 694 - 698
Terada, S. et al., *Cytotechnology* **1997,** *25*, 17-23
Tey, B. T. et al., *Biotechnol Bioeng* **2000a,** *68*, 31-43
Tey, B. T. et al., *J Biotechnol* **2000b,** *79*, 147-159
Tusjimoto, Y., *Genes to Cell* **1998,** *3*, 697 - 707
Urlaub, G. et al., *Cell* **1983,** *33*, 405-412
Werner, R. G. et al., *Arzneim.-Forsch.*/*Drug.Res.* **1998,** *48*, 870-880
Wigler, M. et al., *Proc Natl Acad Sci USA* **1980,** *77*, 3567 - 3570
Wirth and Hauser, Genetic Engineering of Animals Cells, in: Biotechnology Vol. 2, Pühler ed., VCH, Weinheim 663-744
Wu et al., eds., Recombinant DNA Methodology, Academic Press (1989)
Wyllie, A.H. et al., *Int Rev Cytol* **1980,** *68*, *251-* 306
Zanghi, J. A. et al., *Biotechnol Bioeng* **1999,** *64*, 108-119
Zanghi, J. A. et al., *Biotechnol Prog* **2000,** *16*, 319-325

SEQUENCE LISTING

<110> Boehringer Ingelheim Pharma KG

<120> Host cells having improved cell survival properties and
      methods to generate such cells.

<130> sequence protocol case 1-1314

<140>
<141>

<160> 2

<170> PatentIn Ver. 2.1

<210> 1
<211> 926
<212> DNA
<213> homo sapiens

<300>

<400> SEQ ID NO:1
gaatctcttt ctctcccttc agaatcttat cttggctttg gatcttagaa gagaatcact 60
aaccagagac gagactcagt gagtgagcag gtgttttgga caatggactg gttgagccca 120
tccctattat aaaaatgtct cagagcaacc gggagctggt ggttgacttt ctctcctaca 180
agctttccca gaaaggatac agctggagtc agtttagtga tgtggaagag aacaggactg 240

aggccccaga agggactgaa tcggagatgg agacccccag tgccatcaat ggcaacccat 300
cctggcacct ggcagacagc cccgcggtga atggagccac tgcgcacagc agcagtttgg 360
atgcccggga ggtgatcccc atggcagcag taaagcaagc gctgagggag gcaggcgacg 420
agtttgaact gcggtaccgg cgggcattca gtgacctgac atcccagctc cacatcaccc 480
cagggacagc atatcagagc tttgaacagg tagtgaatga actcttccgg gatggggtaa 540
actggggtcg cattgtggcc tttttctcct tcggcggggc actgtgcgtg gaaagcgtag 600
acaaggagat gcaggtattg gtgagtcgga tcgcagcttg gatggccact tacctgaatg 660
accacctaga gccttggatc caggagaacg gcggctggga tacttttgtg gaactctatg 720
ggaacaatgc agcagccgag agccgaaagg gccaggaacg cttcaaccgc tggttcctga 780
cgggcatgac tgtggccggc gtggttctgc tgggctcact cttcagtcgg aaatgaccag 840
acactgacca tccactctac cctcccaccc ccttctctgc tccaccacat cctccgtcca 900
gccgccattg ccaccaggag aacccg 926

<210> 2
<211> 911
<212> DNA
<213> homo sapiens

<400> SEQ ID NO:2
tgattgaaga caccccctcg tccaagaatg caaagcacat ccaataaaat agctggatta 60
taactcctct tctttctctg ggggccgtgg ggtgggagct ggggcgagag gtgccgttgg 120
cccccgttgc ttttcctctg ggaaggatgg cgcacgctgg gagaacgggg tacgacaacc 180
gggagatagt gatgaagtac atccattata agctgtcgca gaggggctac gagtgggatg 240
cgggagatgt gggcgccgcg cccccggggg ccgcccccgc accgggcatc ttctcctccc 300
agcccgggca cacgccccat ccagccgcat cccgcgaccc ggtcgccagg acctcgccgc 360
tgcagacccc ggctgccccc ggcgccgccg cggggcctgc gctcagcccg gtgccacctg 420
tggtccacct ggccctccgc caagccggcg acgacttctc ccgccgctac cgcggcgact 480
tcgccgagat gtccagccag ctgcacctga cgcccttcac cgcgcgggga cgctttgcca 540
cggtggtgga ggagctcttc agggacgggg tgaactgggg gaggattgtg gccttctttg 600
agttcggtgg ggtcatgtgt gtggagagcg tcaaccggga gatgtcgccc ctggtggaca 660
acatcgccct gtggatgact gagtacctga accggcacct gcacacctgg atccaggata 720
acggaggctg ggtaggtgca tctggtgatg tgagtctggg ctgaggccac aggtccgaga 780
tcgggggttg gagtgcgggt gggctcctgg gcaatgggag gctgtggagc cggcgaaata 840
aaatcagagt tgttgcttcc cggcgtgtcc ctacctcctc ctctggacaa agcgttcact 900
cccaacctga c 911

## Claims

1. A hamster host cell genetically modified by introducing nucleic acid sequences that encode for an anti-apoptosis gene, a selectable amplifiable marker gene, and at least one gene of interest.

2. A host cell of claim 1, wherein the host cell is a Chinese Hamster Ovary (CHO) cell or a Baby Hamster Kidney (BHK) cell.

3. A host cell according to claim 1 or 2, wherein the host cell is a CHO-DG44, CHO-K1, CHO-DUKX, CHO-DUKX B1, CHO Pro-5, V79, B14AF28-G3, BHK-21, BHK TK⁻, HaK, or BHK-21(2254-62.2) cell, or the progeny of any of such cell line.

4. A murine myeloma cell genetically modified by introducing nucleic acid sequences that encode for an anti-apoptosis gene, a selectable amplifiable marker gene, and at least one gene of interest.

5. A host cell of claim 4, wherein the host cell is a NS0 or SP2/0-Ag14 cell, or the progeny of any of such cell line.

6. A host cell according to any of claims I to 5, wherein the anti-apoptosis gene encodes for a member of the Bc1-2 superfamily that can act as cell death repressor.

7. A host cell according to any one of claims 1 to 6, wherein the anti-apoptosis gene encodes for BCL-xL, BCL-2, BCL-w, BFL-1, A1, MCL-1, BOO, BRAG-1, NR-13, CDN-1, CDN-2, CDN-3, BHRF-1, LMW5-HL or CED-9.

8. A host cell according to any one of claims 1 to 6, wherein the anti-apoptosis gene encodes for BCL-xL or BCL-2.

9. A host cell according to any one of claims 1 to 7, wherein the anti-apoptosis gene has the sequence of SEQ ID NO:1 or SEQ ID NO:2.

10. A host cell according to any one of claim 1 to 7, encoding for BCL-xL.

11. A host cell according to any one of claims 1 to 7, wherein the anti-apoptosis gene has the sequence of SEQ ID NO:1

12. A host cell according to any one of claims 1 to 9, wherein the selectable amplifiable marker gene encodes for dhydrofolate reductase (DHFR), glutamine synthetase, CAD, adenosine deaminase, adenylate deaminase, UMP synthetase, IMP 5'-dehydrogenase, xanthine guanine phosphoribosyl transferase, HGPRTase, thymidine kinase, thymidylate synthetase, P glycoprotein 170, ribonucleotide reductase, asparagine synthetase, arginosuccinate synthetase, ornithine decarboxylase, HMG CoA reductase, acetylglucosaminyl transferase, threonyl-tRNA synthetase or Na⁺K⁺-ATPase.

13. A host cell according to any one of claims 1 to 12, wherein the anti-apoptosis gene encodes for BCL-xL and the selectable amplifiable marker gene for DHFR, glutamine synthetase, CAD, adenosine deaminase, adenylate deaminase, UMP synthetase, IMP 5'-dehydrogenase, xanthine guanine phosphoribosyl transferase, HGPRTase, thymidine kinase, thymidylate synthetase, P glycoprotein 170, ribonucleotide reductase, asparagine synthetase, arginosuccinate synthetase, omithine decarboxylase, HMG CoA reductase, acetylglucosaminyl transferase, threonyl-tRNA synthetase or Na⁺K⁺-ATPase

14. A host cell according to any of claims 1 to 12, wherein the anti-apoptosis gene encodes for BCL-xL and the selectable amplifiable marker gene for DHFR.

15. A host cell according to any of claims 1 to 14, wherein the anti-apoptosis gene, the selectable amplifiable marker gene and the gene(s) of interest are operatively linked to at least one regulatory sequence allowing for expression of said genes.

16. A method of expressing an anti-apoptosis gene, an selectable amplifiable marker gene and at least one gene of interest in a mammalian host cell comprising:
(a) introducing into a hamster host cell population the nucleic acid sequences that encode for an anti-apoptosis gene, a selectable amplifiable marker gene, and a gene(s) of interest,
wherein said genes are operatively linked to at least one regulatory sequence allowing for expression of said genes;
(b) cultivating said host cell population under conditions where said genes are expressed.

17. A method of generating mammalian host cells showing an enhanced expression level of an anti-apoptosis gene comprising:
(a) introducing into a mammalian host cell population nucleic acid sequences that encode for an anti-apoptosis gene, a selectable amplifiable marker gene, and optionally at least one gene of interest, wherein said genes are operatively linked to at least one regulatory sequence allowing for expression of said genes;
(b) cultivating said cell population under conditions where at least said selectable amplifiable marker gene and said anti-apoptosis gene are expressed, and which are favourable for obtaining multiple copies at least of the anti-apoptosis gene;
(c) selecting cells from the cell population that incorporate multiple copies at least of the anti-apoptosis gene.

18. The method of claim 17, wherein the gene(s) of interest is introduced into the mammalian host cell population.

19. The method of any one of claims 16 to 18, wherein the mammalian host cells are murine myeloma or hamster cells.

20. The method of claim 19, wherein the hamster cells are Chinese Hamster Ovary (CHO) cells or Baby Hamster Kidney (BHK) cells.

21. The method of any one of claims 16 to 18, wherein the mammalian host cell is NS0 or SP2/0-Ag14 cell.

22. The method of any one of claims 15 to 19, wherein the anti-apoptosis gene encodes for BCL-xL, BCL-2, BCL-w, BFL-1, A1, MCL-1, BOO, BRAG-1, NR-13, CDN-1, CDN-2, CDN-3, BHRF-1, LMW5-HL or CED-9.

23. The method of any one of claims 16 to 22, wherein the anti-apoptosis gene encodes for BCL-xL or BCL-2.

24. The method of any one of claims 16 to 22, wherein the anti-apoptosis gene has the sequence of SEQ ID NO:1 or SEQ ID NO:2.

25. The method of any one of claims 16 to 22, wherein the anti-apoptosis gene encodes for BCL-xL.

26. The method of any one of claims 16 to 22, wherein the anti-apoptosis gene has the sequence of SEQ ID NO:1

27. The method of any one of claims 16 to 26, wherein the selectable amplifiable marker gene encodes for the dihydrofolate reductase (DHFR), glutamine synthetase, CAD, adenosine deaminase, adenylate deaminase, UMP synthetase, IMP 5'-dehydrogenase, xanthine guanine phosphoribosyl transferase, HGPRTase, thymidine kinase, thymidylate synthetase, P glycoprotein 170, ribonucleotide reductase, asparagine synthetase, arginosuccinate synthetase, ornithine decarboxylase, HMG CoA reductase, acetylglucosaminyl transferase, threonyl-tRNA synthetase or Na⁺K⁺-ATPase.

28. The method of any one of claims 16 to 21, wherein the anti-apoptosis gene encodes for BCL-xL and the selectable amplifiable marker gene for DHFR.

29. A method of generating a mammalian host cell comprising:
(a) introducing into a mammalian host cell population an anti-apoptosis gene and the DHFR gene;
(b) amplifying the anti-apoptosis gene in the presence of methotrexate.

30. The method of claim 29, wherein the anti-apoptosis gene encodes for BC1-2 or BCl1-xL.

31. The method of claim 29, wherein the anti-apoptosis gene encodes for BCl-xL.

32. The method of any of claims 29 to 31, wherein the host cells are murine myeloma or hamster cells.

33. The method of any of claims 29 to 31, wherein the hamster cells are Chinese Hamster Ovary (CHO) cells or Baby Hamster Kidney (BHK) cells.

34. Host cells obtainable by a method of any one of claims 16 to 33.

35. A method for inhibiting or delaying cell death in a host cell, comprising cultivating host cells according to any of claims 1 to 15 or 34 under conditions where at least the anti-apoptosis gene is expressed in such that cell death is inhibited or delayed in said host cells.

36. The method according to claim 35, wherein the cell death is caused by programmed cell death.

37. The method of claim 35, wherein the cell death is caused by apoptosis.

38. The method according to any one of claims 16 to 33 or 35 to 37, wherein the cells are cultivated in a serum and/or protein free culture medium.

39. A process for producing a protein of interest in a host cell, comprising:
(a) cultivating cells according to any of claims 1 to 15 or 34 under conditions which are favourable for the expression of the anti-apoptosis gene and the gene(s) of interest;
(b) isolating the protein of interest from the cells and/or the cell culture supernatant.

40. Use of a cell according to any of claims 1 to 15 or 34 for production of at least one protein encoded by a gene of interest.

41. A host cell comprising at least 5 copies of a heterologous anti-apoptosis gene.

42. A host cell comprising at least 10 copies of a heterologous anti-apoptosis gene.

43. A host cell comprising at least 20 copies of a heterologous anti-apoptosis gene.

44. A host cell comprising at least 50 copies of a heterologous anti-apoptosis gene.

45. A host cell comprising at least 100 copies of a heterologous anti-apoptosis gene.

46. A host cell according to any of claims 37 to 41, wherein the anti-apoptosis gene encodes for BCL-xL, BCL-2, BCL-w, BFL-1, A1, MCL-1, BOO, BRAG-1, NR-13, CDN-1, CDN-2, CDN-3, BHRF-1, LMW5-HL or CED-9.

47. A host cell according to any of claims 41 to 45, wherein the anti-apoptosis gene is BCL-xL or BCL-2.

48. A host cell according to any of claims 41 to 45, wherein the anti-apoptosis gene has the sequence of of SEQ ID NO:1 or SEQ ID NO:2.

49. A host cell according to any of claims 41 to 45, wherein the anti-apoptosis gene is BCL-xL.

50. A host cell according to any of claims 41 to 49, wherein the host cell is a murine hybridoma or hamster cell.

51. A host cell according to any of claims 41 to 49, wherein the host cell is a Chinese Hamster Ovary (CHO) cell or a Baby Hamster Kidney (BHK) cell.

52. A host cell according to claim 50, wherein the murine myeloma cell is a NS0 or SP2/0-Ag14 cell, or the progeny of any of such cell line.

53. A host cell according to any of claims 41 to 49, wherein the host cell is a CHO-DG44, CHO-K1, CHO-DUKX, CHO-DUKX B1, CHO Pro-5, V79, B14AF28-G3, BHK-21, BHK TK⁻, HaK, BHK-21(2254-62.2), or the progeny of any of such cell line.

54. A host cell according to any of claims 41 to 53, further comprising at least one heterologous gene of interest.

55. A host cell according to any of claims 1 to 15, or 34 comprising at least 5 copies of the heterologous anti-apoptosis gene.

56. A host cell according to any of claims 1 to 15, or 34 comprising at least 10 copies of the heterologous anti-apoptosis gene.

57. A host cell according to any of claims 1 to 15, or 34 comprising at least 20 copies of the heterologous anti-apoptosis gene.

58. A host cell according to any of claims 1 to 15, or 33 comprising at least 50 copies of the heterologous anti-apoptosis gene.

59. A host cell according to any of claims 1 to 15, or 34 comprising at least 100 copies of the heterologous anti-apoptosis gene.
